(19) Europäisches Patentamt
European Patent Office
Office européen des brevets

(11) EP 1 321 145 A1

(12) **EUROPEAN PATENT APPLICATION**
published in accordance with Art. 158(3) EPC

(43) Date of publication:
**25.06.2003 Bulletin 2003/26**

(21) Application number: **01984400.0**

(22) Date of filing: **25.07.2001**

(51) Int Cl.[7]: **A61K 31/56**, A61K 35/78, A61P 35/00, C07J 53/00

(86) International application number:
**PCT/JP01/06393**

(87) International publication number:
**WO 02/009719 (07.02.2002 Gazette 2002/06)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **31.07.2000 JP 2000230254**
**30.11.2000 JP 2000366297**

(71) Applicant: **The Nisshin Oillio, Ltd.**
**Tokyo 104-0033 (JP)**

(72) Inventors:
- **KUNO, Noriyasu,**
**c/o THE NISSHIN OIL MILLS, LTD.**
**Yokosuka-shi, Kanagawa 239-0832 (JP)**
- **SHINOHARA, Gou,**
**c/o THE NISSHIN OIL MILLS, LTD.**
**Yokosuka-shi, Kanagawa 239-0832 (JP)**
- **INUI, Tosiyuki,c/o THE NISSHIN OIL MILLS, LTD.**
**Yokosuka-shi, Kanagawa 239-0832 (JP)**

(74) Representative: **Bawden, Peter Charles et al**
**Bawden & Associates,**
**4 The Gatehouse,**
**2 High Street**
**Harpenden, Hertfordshire AL5 2TH (GB)**

(54) **ANTITUMOR AGENTS**

(57) The present invention relates to an antitumor agent, which comprises, as an effective component, a compound selected from the group consisting of maslinic acid, erythrodiol, uvaol, betulinic acid, betulin and physiologically acceptable salts thereof or derivatives thereof.

EP 1 321 145 A1

## Description

### Background of the Invention

The present invention relates to an orally and/or parenterally administeredantitumor agent comprising, as an effective component, at least one member selected from the group consisting of specific 5-membered ring-containing triterpenes and physiologically acceptable salts thereof or derivatives thereof.

Recently, the mortality rate of cancer or tumor has basically been apt to increase in Japan (in 1997), while it has basically been switched off to reducing tendency, at long last, in the United States of America (in 1995), but the cancer or tumor has still taken the second place as the cause of death.

Contrary to this, the mechanism of the cancer development has been elucidated in the molecular level due to the recent intensive and extensive investigations and studies. The fruits of these efforts or studies make it dear that various chemical substances can control various steps of the cancer-developing processes and they have been employed as pharmaceutical agents having antitumor actions.

Theseantitumor agents show their effects in accordance with different mechanisms, respectively and therefore, they permit effective treatments by successfully combining them. Moreover, if a drug has continuously been used, it has been pointed out that a problem of so-called tolerance arises. If taking into consideration the foregoing, the existence of a wide variety ofantitumor agents is quite desirable.

Furthermore, there have conventionally been used various kinds of chemical substances asantitumor agents possessing antitumor actions. They have, on the one hand, strong antitumor actions, but on the other hand, they have such a side effect that they have harmful or injurious actions on not only the tumor cells, but also the normal cells. For this reason, it is quite obvious that desirableantitumor agents should have a lower cytotoxicity and high safety.

On the other hand, the 5-membered ring-containing triterpenes belong to a kind of triterpenes, they are usually 5-membered ring-containing compounds each consisting of six isoprene units in the molecule and the number of carbon atoms thereof is fundamentally 30, but the number of carbon atoms included in the triterpenes may vary through the rearrangement, oxidation, elimination or alkylation in the processes for the biosynthesis of the same. Moreover, they are in general classified on the basis of their skeletons and examples thereof include oleanane type triterpenes, ursane type triterpenes, lupane type triterpenes, hopane type triterpenes, serratane type triterpenes, friedelane type triterpenes, taraxerane type triterpenes, taraxastane type triterpenes, multi-furolane type triterpenes and germanicane type triterpenes.

They in general have an anti-inflammatory effect and an anti-carcinogenic promoter activity and therefore, it has been known that they may be used as prophylactic medicine (Bulletin of the Society of Oil Chemistry in Japan, 2000, 49: 571). Moreover, in this respect, it has already been known that oleanolic acid, erythrodiol, glycyrrhetic acid or the like have a carcinogenic promoter-inhibitory activity, as well (Cancer Letters, 1986, 30: 143-151 or Japanese Un-Examined Patent Publication (hereunder referred to as "J.P. KOKAI") No. Sho 63-57519). With respect to tumor cells, which have already been developed, ursolic acid is known to have a cell-metastasis-inhibitory effect although the tumor cell-proliferation-inhibitory effect and tumor cell-killing (or necrosis) action have not yet been confirmed (J. P. KOKAI No. Hei 9-143076). Up to date, however, it has never been known that specific 5-membered ring-containing triterpenes and physiologically acceptable salts thereof or derivatives thereof including those, which have been known to have carcinogenic promoter- inhibitory activity possess, for instance, a tumor cell-proliferation-inhibitory effect, a tumor cell-necrosis effect and a tumor cell-metastasis-inhibitory effect.

### Disclosure of the Invention

It is an object of the present invention to provide an orally and/or parenterally administered anti-v agent comprising, as an effective component, at least one member selected from the group consisting of specific 5-membered ring-containing triterpenes and physiologically acceptable salts thereof or derivatives thereof and a method for using the same. More particularly, it is an object of the present invention to provide an antitumor agent having a tumor cell-proliferation-inhibitory effect, a tumor cell-necrosis effect and a tumor cell-metastasis-inhibitory effect as well as a method for using the same.

The inventors of this invention have conducted various studies to achieve the foregoing objects, have found that specific 5-membered ring-containing triterpenes and physiologically acceptable salts thereof or derivatives thereof possess excellent tumor cell-proliferation-inhibitory, tumor cell-necrosis (or killing) and tumor cell-metastasis- inhibitory effects and have thus completed the present invention.

More specifically, the present invention relates to anantitumor agent comprising, as an effective component, one or at least two members selected from the group consisting of maslinic acid, erythrodiol, ursolic acid, uvaol, betulinic acid, betulin and physiologically acceptable salts thereof or derivatives thereof, preferably relates to anantitumor agent having a tumor cell-proliferation-inhibitory effect, preferably relates to anantitumor agent having a tumor cell-lulling

effect and preferably relates to anantitumor agent having a tumor cell-metastasis-inhibitory effect.

In addition, the present invention preferably relates to anantitumor agent comprising, as an effective component, maslinic acid and/or a physiologically acceptable salt thereof, preferably relates to anantitumor agent having a tumor cell-proliferation-inhibitory effect, preferably relates to anantitumor agent having a tumor cell-killing effect and preferably relates to anantitumor agent having a tumor cell-metastasis-inhibitory effect.

In this connection, the maslinic acid, erythrodiol, ursolic acid, uvaol, betulinic acid and betulin used in the present invention may be those extracted from naturally occurring raw materials or may be any commercially available reagents. In addition, the physiologically acceptable salts and derivatives thereof may likewise be those extracted from naturally occurring raw materials or may be any synthesized products prepared through synthetic reactions using such extract as raw materials. In particular, it is preferred to use those isolated from naturally occurring raw materials.

Moreover, the present invention relates to a method of using, as anantitumor agent, at least one member selected from the group consisting of maslinic acid, erythrodiol, ursolic acid, uvaol, betulinic acid, betulin and physiologically acceptable salts thereof or derivatives thereof. In particular, the present invention likewise relates to a method of using at least one member selected from the group consisting of maslinic acid, erythrodiol, ursolic acid, uvaol, betulinic acid, betulin and physiologically acceptable salts thereof or derivatives thereof, for achieving at least one of the following effects: a tumor cell-proliferation-inhibitory effect, a tumor cell-killing effect and a tumor cell-metastasis-inhibitory effect, as well as a method of using at least one member selected from the group consisting of ursolic acid, and physiologically acceptable salts thereof or derivatives thereof, for achieving tumor cell-proliferation-inhibitory and/or tumor cell-killing effects.

The method of using the same is not restricted to any specific one, but the antitumor agent may directly be applied to the external and/or internal tumor lesions of human bodies to thus accomplish any desired effect. Moreover, the similar effects can likewise be expected by administering the agent through, for instance, oral route or through injection.

Accordingly, the present invention also relates to a method for inhibiting tumor cell-proliferation or killing tumor cells, which comprises the step of bringing a compound selected from the group consisting of maslinic acid, erythrodiol, ursolic acid, uvaol, betulinic acid, betulin and physiologically acceptable salts thereof or derivatives thereof as an effective component into contact with tumor cells and/or allowing the compound to penetrate into tumor cells.

In addition, the present invention also relates to a method for inhibiting the metastasis of tumor cells, which comprises the step of bringing a compound selected from the group consisting of maslinic acid, erythrodiol, uvaol, betulinic acid, betulin and physiologically acceptable salts thereof or derivatives thereof as an effective component into contact with tumor cells and/or allowing the compound to penetrate into tumor cells.

## Best Mode for Carrying Out the Invention

The present invention relates to anantitumor agent comprising, as an effective component, at least one member selected from the group consisting of maslinic acid, erythrodiol, ursolic acid, uvaol, betulinic acid, betulin and physiologically acceptable salts thereof or derivatives thereof and in particular, to anantitumor agent whoseantitumor effect is a tumor cell-proliferation-inhibitory effect, a tumor cell-killing effect and/or a tumor cell-metastasis-inhibitory effect. The term "comprising, as an effective component" used herein means that anantitumor agent contains an effective component in such an amount sufficient for achieving the desired tumor cell-proliferation-inhibitory, tumor cell-killing and/or tumor cell-metastasis-inhibitory effects.

Moreover, the present invention preferably relates to anantitumor agent comprising, as an effective component, maslinic acid and/or a physiologically acceptable salt thereof.

Maslinic acid is a compound present in, for instance, olives, hops, peppermints, pomegranates, clove, sage and jujubes and the physiologically acceptable salts thereof are those derived from the group: -COOH in the chemical formula (I) and the kinds of salts are not restricted to specific ones and may be those commonly used in foods and beverages or medical compositions.

The term "comprising, as an effective component" used herein means that anantitumor agent contains an effective component in such an amount sufficient for achieving the desired tumor cell-proliferation-inhibitory, tumor cell-killing and/or tumor cell-metastasis-inhibitory effects, as has been described above.

The maslinic acid, erythrodiol, ursolic acid, uvaol, betulinic acid, betulin and physiologically acceptable salts thereof or derivatives thereof incorporated into the antitumor agent of the present invention possess a tumor cell-proliferation- inhibitory effect, they have a tumor cell-killing effect and they also show an effect of controlling or inhibiting any metastasis of tumor cells.

In this respect, the maslinic acid, erythrodiol, ursolic acid, betulinic acid and betulin possess an anti-carcinogenic promoter (antitumor promoter) activity and therefore, it would be expected that they could control the carcinogenesis (or oncogenesis) or inhibit the development of any tumor cells. Moreover, the antitumor agent of the present invention has an effect of suppressing the tumor cell-proliferation; killing the tumor cells; and inhibiting the metastasis

thereof even in an undetectable level and in other words, the agent has an effect of extinguishing tumor in an invisible level through the ordinary intake of the agent. For this reason, the agent of the present invention can likewise be used as a prophylactic agent. Moreover, the antitumor agent of the present invention can be used as a therapeutic agent for inhibiting any tumor cell-proliferation; killing the cells and/or inhibiting any metastasis of the tumor cell, immediately after the development of the tumor cell or as an agent for inhibiting the progress of the tumor and extinguishing the tumor. In addition, theseantitumor agents can directly be applied to the external and/or internal tumor lesions of human bodies to thus accomplish any desired effect. Moreover, the similar effects can likewise be expected by administering the agent through, for instance, the oral route or through injection.

The tumors to which the antitumor agent of the present invention can be applied include swellings and true tumors including benign and malignant tumors. Specific examples of such tumors are gliomas such as astrocytoma, glioblastoma, medulloblastoma, oligodendroglioma, ependymoma and choroid plexus papilloma; cerebral tumors such as meningioma, pituitary adenoma, neurioma, congenital tumor, metastatic cerebral tumor; squamous cell carcinoma, lymphoma, a variety of adenomas and pharyngeal cancers resulted from these adenomas such as epipharyngeal cancer, mesopharyngeal cancer and hypopharyngeal cancer; laryngeal cancer, thymoma; mesothelioma such as pleural mesothelioma, peritoneal mesothelioma and pericardial mesothelioma; breast cancers such as thoracic duct cancer, lobular carcinoma and papillary cancer; lung cancers such as small cell carcinoma, adenocarcinoma, squamous cell carcinoma, large cell carcinoma and adenosquamous carcinoma; gastric carcinoma; esophageal carcinomas such as cervical esophageal carcinomas, thoracic esophageal carcinomas and abdominal esophageal carcinomas; carcinomas of large intestine such as rectal carcinoma, S-like (sigmoidal) colon carcinoma, ascending colon carcinoma, lateral colon carcinoma, cecum carcinoma and descending colon carcinoma; hepatomas such as hepatocellular carcinoma, intrahepatic hepatic duct carcinoma, hepatocellular blastoma and hepatic duct cystadenocarcinoma; pancreatic carcinoma; pancreatic hormone-dependent tumors such as insulinoma, gastrinoma, VIP-producing adenoma, extrahepatic hepatic duct carcinoma, hepatic capsular carcinoma, perial carcinoma, renal pelvic and uretal carcinoma; urethral carcinoma; renal cancers such as renal cell carcinoma (Grawitz tumor), Wilms' tumor (nephroblastoma) and renal angiomyolipoma; testicular cancers or germ cell tumors such as seminoma, embryonal carcinoma, vitellicle tumor, choriocarcinoma and teratoma; prostatic cancer, bladder cancer, carcinoma of vulva; hysterocarcinomas such as carcinoma of uterine cervix, uterine corpus cancer and solenoma; hysteromyoma, uterine sarcoma, villous diseases, carcinoma of vagina; ovarian germ cell tumors such as dysgerminoma, vitellicle tumor, premature teratoma, dermoidal cancer and ovarian tumors such as ovarian cancer; melanomas such as nevocyte and melanoma; skin lymphomas such as mycosis fungoides, skin cancers such as endoepidermal cancers resulted from skin cancers, prodrome or the like and spinocellular cancer, soft tissue sarcomas such as fibrous histiocytomatosis, liposarcoma, rhabdomyosarcoma, leiomyosarcoma, synovial sarcoma, sarcoma fibroplasticum (fibrosarcoma), neurioma, hemangiosarcoma, fibrosarcoma, neurofibrosarcoma, perithelioma (hemangiopericytoma) and alveolar soft part sarcoma, lymphomas such as Hodgkin lymphoma and non-Hodgkin lymphoma, myeloma, plasmacytoma, acute myelocytic (myeloid) leukemia and chronic myeloid leukemia, leukemia such as adult T-cell leukemic lymphoma and chronic lymphocytic leukemia, chronic myeloproliferative diseases such as true plethora, essential thrombocythemia and idiopathic myelofibrosis, lymph node enlargement (or swelling), tumor of pleural effusion, ascitic tumor, other various kinds of adenomas, lipoma, fibroma, hemangeoma, myoma, fibromyoma and endothelioma.

The present invention relates to anantitumor agent comprising, as an effective component, one or at least two members selected from the group consisting of 5-membered ring-containing triterpenes and physiologically acceptable salts thereof or derivatives thereof. In this connection, the 5-membered ring-containing triterpene is a kind of triterpene, usually a 5-membered ring-containing compound consisting of six isoprene units in the molecule and the number of carbon atoms thereof is fundamentally 30, but the number of carbon atoms included in the triterpenes may vary through the rearrangement, oxidation, elimination or alkylation in the processes for the biosynthesis of the same.

These substances may be obtained from naturally occurring plants or may artificially be prepared. Moreover, commercially available ones may likewise favorably be used in the invention. Among the foregoing, preferred are those obtained from naturally occurring plants. For instance, maslinic acid and/or physiologically acceptable salts thereof can suitably be prepared from olive plants and/or products produced in the olive oil production processes. Therefore, preferred embodiments herein include anantitumor agent, a tumor cell-proliferation-inhibitory agent, a tumor cell-killing agent or a tumor cell-metastasis-inhibitory agent, which comprises maslinic acid and/or physiologically acceptable salts thereof thus obtained. In this respect, the term "olive" used in the specification means olive plants and/or products produced in the olive oil production processes.

The 5-membered ring-containing triterpenes are in general classified on the basis of their skeletons. Examples thereof include oleanane type triterpenes, ursane type triterpenes, lupane type triterpenes, hopane type triterpenes, serratane type triterpenes, friedelane type triterpenes, taraxerane type triterpenes, taraxastane type triterpenes, multifurolane type triterpenes and germanicane type triterpenes.

The inventors of this invention have found that among these substances, specific 5-membered ring-containing triterpenes, or maslinic acid, erythrodiol as oleanane type triterpene, ursolic acid, uvaol as ursane type triterpene and

betulinic acid, betulin as lupine type triterpene have excellent antitumor effects such as a tumor cell-proliferation-inhibitory effect, a tumor cell-killing effect and a tumor cell-metastasis- inhibitory effect and have thus completed the present invention. At the same time, the inventors have also found that oleanolic acid, β -amyrin as oleanane type triterpene, α -amyrin as ursane type triterpene and lupeol as lupine type triterpene, whose skeletons are similar to those listed above, do not show anyantitumor effects such as a tumor cell-proliferation-inhibitory effect, a tumor cell-killing effect and a tumor cell-metastasis-inhibitory effect, at all. In other words, only specific substances among the 5-membered ring-containing triterpenes show the intended effects of the present invention or antitumor effects and compounds whose structures are similar to those of the substances having desired antitumor effects and found in the present invention do not always show desiredantitumor effect. For instance, oleanolic acid and maslinic acid are quite resemble in their structures, but theantitumor effects thereof are different from one another to such an extent that maslinic acid is better beyond comparison. The present invention has thus found out such substances having excellent antitumor effects, which are randomly present in the nature.

The present invention relates to anantitumor agent comprising, as an effective component, one or at least two member selected from the group consisting of maslinic acid, erythrodiol, ursolic acid, uvaol, betulinic acid, betulin and physiologically acceptable salts thereof or derivatives thereof, preferably relates to anantitumor agent possessing a tumor cell-proliferation-inhibitory effect, preferably relates to anantitumor agent possessing a tumor cell-killing effect and preferably relates to anantitumor agent possessing a tumor cell-metastasis-inhibitory effect.

In this respect, the term "physiologically acceptable salts" used herein means, in particular, those derived from the carboxyl groups of specific 5-membered ring- containing triterpenes (partial structure thereof: -COOX; X represents an arbitrarily selected cationic substance) and in the present invention, these salts are not restricted to specific ones inasmuch as they are currently used in foods and beverages and medical or pharmaceutical compositions. Specific examples thereof include alkali metal salts such as sodium, potassium and lithium salts; alkaline earth metal salts such as calcium, magnesium, barium and zinc salts; alkylamine salts such as salts with, for instance, ammonia, methylamine, dimethylamine, trimethylamine, ethylamine, diethylamine, triethylamine, propylamine, butylamine, tetrabutylamine, pentylamine and hexylamine; alkanolamine salts such as salts with, for instance, ethanolamine, diethanolamine, triethanolamine, propanolamine, dipropanolamine, isopropanolamine and diisopropanolamine; salts with other organic amines such as piperazine and piperidine; and salts with basic amino acids such as lysine, alginine, histidine and tryptophane. On the whole, these salts have solubility in water higher than that of the original specific 5-membered ring-containing triterpenes and therefore, the salts are preferably used, in particular, in aqueous systems in the present invention.

Moreover, the term "derivatives" used herein means those capable of being biochemically or artificially formed and in the present invention, they are not restricted to specific ones insofar as they can biochemically or artificially be formed. Examples thereof include derivatives having alcohol ester groups, derivatives having fatty acid ester groups, derivatives having alkoxy groups, derivatives having alkoxymethyl groups, or glycosides. Among these, derivatives having alcohol ester groups, derivatives having fatty acid ester groups, derivatives having alkoxy groups and derivatives having alkoxymethyl groups have solubility in oil higher than that of the original specific 5-membered ring-containing triterpenes and therefore, these derivatives are preferably used, in particular, in oily systems in the present invention, while the glycosides have solubility in water higher than that of the original specific 5-membered ring-containing triterpenes and therefore, the glycosides are preferably used, in particular, in aqueous systems in the present invention.

Apart of these derivatives are also existing in the nature and thus they can be isolated from the naturally occurring raw material or they can likewise be formed artificially.

Thus, maslinic acid, erythrodiol, ursolic acid, uvaol, betulinic acid and betulin may be improved in their solubility in water or solubility in oil by converting them into physiologically acceptable appropriate salt or derivatives and therefore, the final products may arbitrarily be designed such that they have improved handling ability, quality and desired effects.

The term "alcohol ester group" herein used means a functional group generally formed as a result of the dehydration reaction of carboxyl groups with alcohols (partial structure: -COOR; R represents an arbitrary hydrocarbon functional group). More specifically, the derivatives of the 5-membered ring-containing triterpenes and having alcohol ester groups are, in particular, those formed from the carboxyl groups of the triterpenes with alcohols. In this respect, the alcohols are not restricted to specific ones, but specific examples thereof include methanol, ethanol, n-propanol, isopropanol, allyl alcohol, n-butanol, sec-butanol, tert-butanol, ethylene glycol, trimethylsilyl alcohol, triethylsilyl alcohol, phenol, benzyl alcohol and saccharides. Among these, preferred are derivatives formed from ethanol, triethylsilyl alcohol, methanol, n-propanol, isopropanol and trimethylsilyl alcohol.

The term "fatty acid ester groups" herein used means functional groups generally formed as a result of the dehydration reaction of hydroxyl groups with fatty acids (partial structure: -OCOR; R represents an arbitrary hydrocarbon functional group). More specifically, the derivatives of 5-membered ring-containing triterpenes of the present invention and having fatty acid ester groups are, in particular, those formed through the reaction of hydroxyl groups of the triterpenes with fatty acids. In this respect, the fatty acids usable herein are not particularly limited, but specific

examples thereof are acetic acid, acetic anhydride, propionic acid, butyric acid, isobutyric acid, valeric acid, isovaleric acid, pivalic acid, caproic acid, caprylic acid, capric acid, undecanoic acid, lauric acid, myristic acid, palmitic acid, palmitoleic acid, stearic acid, oleic acid, elaidic acid, vaccenic acid, linoleic acid, linoelaidic acid, linolenic acid, γ -linolenic acid, arachidic acid, arachidonic acid, eicosapentaenoic acid, behenic acid, docosahexaenoic acid, lignoceric acid, cerotic acid, montanoic acid and melissic acid. Among these, preferred are derivatives derived or formed from acetic acid, acetic anhydride, caproic acid, caprylic acid, capric acid, lauric acid, myristic acid, palmitic acid, palmitoleic acid, stearic acid, oleic acid, elaidic acid, linoleic acid, linoelaidic acid, linolenic acid, γ -linolenic acid, arachidic acid, arachidonic acid, eicosapentaenoic acid, behenic acid and docosahexaenoic acid.

The term "alkoxy group" herein used means a functional group formed as a result of the usual dehydration reaction of a hydroxyl group with an alcohol (partial structure: -OR; R represents an arbitrary hydrocarbon functional group). More specifically, the derivatives of 5-membered ring-containing triterpenes and having alkoxy groups in the present invention are, in particular, those capable of being formed from the hydroxyl groups of the triterpenes and alcohols. In this respect, alcohols are not restricted to specific ones, but specific examples thereof are methanol, ethanol, n-propanol, isopropanol, allyl alcohol, n-butanol, sec-butanol, tert-butanol, ethylene glycol, trimethylsilyl alcohol, triethylsilyl alcohol, phenol, benzyl alcohol and saccharides. Among these, preferred are derivatives formed from ethanol, triethylsilyl alcohol, methanol, n-propanol, isopropanol and trimethylsilyl alcohol.

The term "alkoxymethyl groups" herein used means functional groups generally formed as a result of the dehydration reaction of hydroxylmethyl groups with alcohols (partial structure: $-CH_2OR$; R represents an arbitrary hydrocarbon functional group). More specifically, the derivatives of the 5-membered ring-containing triterpenes of the present invention and having alkoxymethyl groups are, in particular, those formed through the reaction of hydroxyl groups of the triterpenes with alcohols. In this respect, the alcohols usable herein are not particularly limited, but specific examples thereof are methanol, ethanol, n-propanol, isopropanol, allyl alcohol, n-butanol, sec-butanol, tert-butanol, ethylene glycol, trimethylsilyl alcohol, triethylsilyl alcohol, phenol, benzyl alcohol and saccharides. Among these, preferred are derivatives formed from ethanol, triethylsilyl alcohol, methanol, n-propanol, isopropanol and trimethylsilyl alcohol.

Moreover, the term "glycosides" used herein means derivatives capable of being formed from, in particular, carboxyl, hydroxyl and hydroxymethyl groups of the 5-membered ring-containing triterpenes among the foregoing derivatives having alcohol ester groups, derivatives having alkoxy groups and derivatives having alkoxymethyl groups, with saccharides (partial structure: -COOR, -OR, $-CH_2OR$; R represents an arbitrary saccharide moiety). In this respect, the saccharides are not particularly restricted, but specific examples thereof are glucose, mannose, galactose, fructose, xylose, arabinose, fucose, rhamnose, glucosamine, galactosamine and glucuronic acid, which may be either α -isomer or β -isomer. Moreover, these glycosides may be derivatives with monosaccharides or oligo saccharides consisting of disaccharides or higher saccharides constituted by any combination of saccharides. Some of these glycosides are commonly found in nature and have been known under the generic name of "saponin". Either of these glycosides may be used in the present invention.

The present invention relates to anantitumor agent comprising, as an effective component, maslinic acid, erythrodiol, ursolic acid, uvaol, betulinic acid, betulin and physiologically acceptable salts thereof or derivatives thereof. In this connection, the passage "comprising, as an effective component" herein used means that the agent comprises each component in an amount sufficient for achieving the desired antitumor effects or a tumor cell-proliferation-inhibitory effect, a tumor cell-killing effect and a tumor cell-metastasis-inhibitory effect. The "amount capable of showing the intended effect" means that the amount of maslinic acid, erythrodiol, ursolic acid, uvaol, betulinic acid, betulin and physiologically acceptable salts thereof or derivatives thereof included in the antitumor agent of the present invention administered to a subject ensures the desired effect in the treatment of tumors.

Regarding the maslinic acid, erythrodiol as oleanane type triterpenes, ursolic acid, uvaol as ursane type triterpenes, betulinic acid, betulin as lupine type triterpenes and physiologically acceptable salts thereof or derivatives thereof, the origins thereof are not restricted to any specific one and usable herein may be those derived from natural products, those artificially synthesized and commercially available ones.

Both maslinic acid and erythrodiol are substances belonging to the oleanane type triterpenes and it has been known that they are present in a variety of plants. When using maslinic acid, erythrodiol, physiologically acceptable salts thereof and/or derivatives thereof in the antitumor agent, the origins thereof are not restricted to specific ones and they may be those isolated from natural resources, those artificially synthesized or commercially available ones.

Maslinic acid is one of oleanane type triterpenes and a compound represented by the following chemical formula (I). It has been known that this substance possesses an anti-inflammatory effect and an anti-histaminic action. These substances are known to exist in the nature, for instance, in olives, hops, peppermints, pomegranates, clove, sage and jujubes. In the antitumor agent of the present invention, the origins of the maslinic acid and physiologically acceptable salts thereof or derivatives thereof are not limited to specific ones and they may be those isolated from natural resources, those artificially synthesized or commercially available ones, but preferred are those derived or isolated from natural resources such as olives, hops, peppermints, pomegranates, dove, sage and jujubes. Particularly

stable sources of supply thereof are, for instance, olive plants grown as raw materials for oils and fats. Olive plants are quite preferred since they are stably and continuously cultivated and they comprise maslinic acid and/or physiologically acceptable salts thereof in high concentrations. Preferably used herein also include those prepared from the products (by-products) obtained in the olive oil-manufacturing processes.

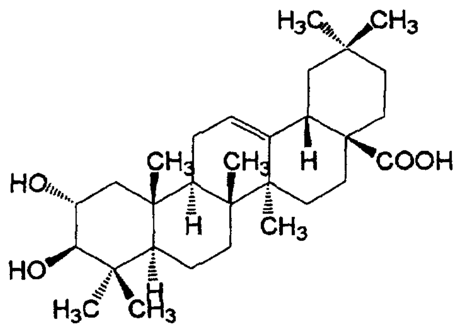

(1)

In the present invention, the foregoing is likewise true for the physiologically acceptable salts and derivatives of maslinic acid. More specifically, the "physiologically acceptable salts" thereof herein means those derived from the -COOH group in the chemical formula (I) and the kinds of salts are not limited to any specific one inasmuch as they are commonly used in foods and beverages and pharmaceutical compositions. Specific examples of maslinic acid salts are sodium maslinate, potassium maslinate, ammonium maslinate, dimethylammonium maslinate, calcium maslinate and magnesium maslinate. Preferred are sodium maslinate and potassium maslinate, among others.

As the derivatives of maslinic acid, for instance, those each derivatized at a single position, there may be listed, for instance, maslinic acid methyl ester, maslinic acid ethyl ester, maslinic acid n-propyl ester, maslinic acid isopropyl ester, maslinic acid n-butyl ester, maslinic acid trimethylsilyl ester, maslinic acid triethylsilyl ester, maslinic acid- β -D-glucopyranosyl ester, maslinic acid- β -D-galactopyranosyl ester, 3- O-acetyl-maslinic acid, 3-O-propionyl-maslinic acid, 3-O-butyryl-maslinic acid, 3-O- valeryl-maslinic acid, 3-O-capryl-maslinic acid, 3-O-lauryl-maslinic acid, 3-O-myristyl- maslinic acid, 3-O-palmityl-maslinic acid, 3-O-palmito-oleyl-maslinic acid, 3-O-stearoyl-maslinic acid, 3-O-oleyl-maslinic acid, 3-O-vaccenyl-maslinic acid, 3-O-linoleyl-maslinic acid, 3-O-linolenyl-maslinic acid, 3-O-arachidyl-maslinic acid, 3-O-arachidonyl-maslinic acid, 3-O-behenyl-maslinic acid, 2-O-acetyl-maslinic acid, 2-O-propionyl-maslinic acid, 2-O-butyryl-maslinic acid, 2-O-valeryl-maslinic acid, 2-O-capryl-maslinic acid, 2-O- lauryl-maslinic acid, 2-O-myristyl-maslinic acid, 2-O-palmityl-maslinic acid, 2-O- palmito-oleyl-maslinic acid, 2-O-stearoyl-maslinic acid, 2-O-oleyl-maslinic acid, 2-O-vaccenyl-maslinic acid, 2-O-linoleyl-maslinic acid, 2-O-linolenyl-maslinic acid, 2-O-arachidyl-maslinic acid, 2-O-arachidonyl-maslinic acid, 2-O-behenyl-maslinic acid, 3-O-methyl-maslinic acid, 3-O-ethyl-maslinic acid, 3-O-t-butyl-maslinic acid, 3-O- trimethylsilyl-maslinic acid, 3-O-triethylsilyl-maslinic acid, 3-O-benzyl-maslinic acid, 3-O- β -D-glucopyranosyl-maslinic acid, 3-O- β -D-galactopyranosyl-maslinic acid, 3-O- β -D-glucuronopyranosyl-maslinic acid, 2-O-methyl-maslinic acid, 3-O-ethyl-maslinic acid, 2-O-t-butyl-maslinic acid, 2-O-trimethylsilyl-maslinic acid, 2-O-triethylsilyl-maslinic acid, 2-O-benzyl-maslinic acid, 2-O- β -D-glucopyranosyl-maslinic acid, 2-O- β -D-galactopyranosyl-maslinic acid and 2-O- β -D-glucuronopyranosyl-maslinic acid.

Among them, particularly preferred are maslinic acid ethyl ester, maslinic acid triethylsilyl ester, 3-O-acetyl-maslinic acid, 2-O-acetyl-maslinic acid, 2-O-triethylsilyl- maslinic acid, 3-O-stearoyl-maslinic acid, 2-O-stearoyl-maslinic acid, maslinic acid- β -D-glucopyranosyl ester, 3-O-oleyl-maslinic acid, 3-O-linoleyl-maslinic acid, 3-O- linolenyl-maslinic acid, 2-O-oleyl-maslinic acid, 2-O-linoleyl-maslinic acid, 2-O- linolenyl-maslinic acid, 3-O- β -D-glucopyranosyl-maslinic acid and 2-O- β -D- glucopyranosyl-maslinic acid. More particularly preferred are maslinic acid ethyl ester, maslinic acid triethylsilyl ester, 3-O-acetyl-maslinic acid, 2-O-acetyl-maslinic acid, 2-O-triethylsilyl- maslinic acid, 3-O-stearoyl-maslinic acid and 2-O-stearoyl-maslinic acid.

The foregoing are examples of the derivatives in which only one group is derivatized, but it is a matter of course that the derivatives may be derivatized at two or more possible and different positions or derivatized with at least two kinds of counterparts. For instance, preferred examples thereof are 2,3-O-diacetyl derivatives, 2,3-O-ditriethylsilyl derivatives and 2,3-distearoyl derivatives of maslinic acid or the foregoing preferred maslinic acid esters. In addition, only glycosides with monosaccharides are listed above, but it is a matter of course that they may be glycosides with di or higher oligosaccharides comprising combinations of a variety of saccharides.

Erythrodiol is one of oleanane type triterpenes, has a structure represented by the following chemical formula (2) and up to this time, it has been known to have effects such as anti-inflammatory action (Planta. Med., 1995, VOL. 61, No.2, pp.182-185). It is known that this substance is present in the nature, for instance, olive, sunflower, common marigold, gum Senegal, red sanders and Litsea lancifolia Vill. In theantitumor agent of the present invention, the origins

of erythrodiol or derivatives thereof are not restricted to any particular one and the erythrodiol or derivatives thereof may be those prepared or isolated from natural resources, artificially synthesized ones or commercially available ones. For instance, preferably used herein are those derived from or isolated from natural resources such as olive, sunflower, common marigold, gum Senegal, red sanders and Litsea lancifolia Vill. In particular, olive is preferred in the invention and more specifically, preferred are those derived or isolated from olive plants and/or products obtained in olive oil-manufacturing processes.

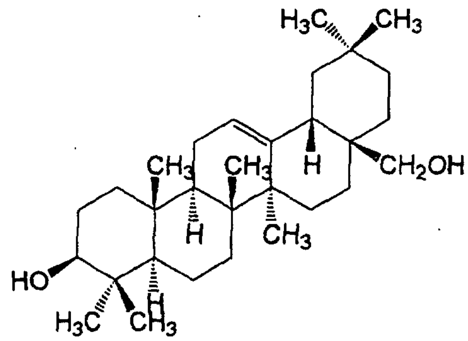

(2)

Regarding erythrodiol, the foregoing is likewise true for the physiologically acceptable salts and derivatives thereof.

In this respect, examples of derivatives thereof each derivatized at a single position include, but not limited to 3-O-acetyl-erythrodiol, 3-O-propionyl-erythrodiol, 3-O-butyryl-erythrodiol, 3-O-valeryl-erythrodiol, 3-O-capryl-erythrodiol, 3-O-lauryl- erythrodiol, 3-O-myristyl-erythrodiol, 3-O-palmityl-erythrodiol, 3-O-palmito-oleyl- erythrodiol, 3-O-stearoyl-erythrodiol, 3-O-oleyl-erythrodiol, 3-O-vaccenyl-erythrodiol, 3-O-linoleyl-erythrodiol, 3-O-linolenyl-erythrodiol, 3-O-arachidyl-erythrodiol, 3-O- arachidonyl-erythrodiol, 3-O-behenyl-erythrodiol, 28-O-acetyl-erythrodiol, 28-O- propionyl-erythrodiol, 28-O-butyryl-erythrodiol, 28-O-valeryl-erythrodiol, 28-O-capryl- erythrodiol, 28-O-lauryl-erythrodiol, 28-O-myristyl-erythrodiol, 28-O-palmityl- erythrodiol, 28-O-palmito-oleyl-erythrodiol, 28-O-stearoyl-erythrodiol, 28-O-oleyl-erythrodiol, 28-O-vaccenyl-erythrodiol, 28-O-linoleyl-erythrodiol, 28-O-linolenyl- erythrodiol, 28-O-arachidyl-erythrodiol, 28-O-arachidonyl-erythrodiol, 28-O-behenyl- erythrodiol, 3-O-methyl-erythrodiol, 3-O-ethyl-erythrodiol, 3-O-t-butyl-erythrodiol, 3-O- trimethylsilyl-erythrodiol, 3-O-triethylsilyl-erythrodiol, 3-O-benzyl-erythrodiol, 28-O- methyl-erythrodiol, 28-O-ethyl-erythrodiol, 28-O-t-butyl-erythrodiol, 28-O- trimethylsilyl-erythrodiol, 28-O-triethylsilyl-erythrodiol, 28-O-benzyl-erythrodiol, 3-O- β -D-glucopyranosyl-erythrodiol, 3-O- β -D-galactopyranosyl-erythrodiol, 3-O- β -D-glucuronopyranosyl-erythrodiol, 28-O- β -D-glucopyranosyl-erythrodiol, 28-O-β -D- galactopyranosyl-erythrodiol and 28-O- β -D-glucuronopyranosyl-erythrodiol.

Among these, particularly preferred are, for instance, 3-O-acetyl-erythrodiol, 28- O-acetyl-erythrodiol, 3-O-oleyl-erythrodiol, 3-O-linoleyl-erythrodiol, 3-O-linolenyl- erythrodiol, 28-O-oleyl-erythrodiol, 28-O-linoleyl-erythrodiol, 28-O-linolenyl-erythrodiol, 3-O- β -D-glucopyranosyl-erythrodiol and 28-O- β -D-glucopyranosyl-erythrodiol.

The foregoing are examples of the derivatives in which only one group is derivatized, but it is a matter of course that the derivatives may be derivatized at two or more possible and different positions or derivatized with at least two kinds of counterparts. There may be listed, for instance, 3,28-O-diacetyl-erythrodiol. In addition, only glycosides with monosaccharides are listed above, but it is likewise a matter of course that they may be glycosides with di or higher oligosaccharides comprising combinations of a variety of saccharides.

Both ursolic acid and uvaol belong to the ursane type triterpene group and they have been known as substances present in a variety of plants. In addition, the foregoing is likewise true for the physiologically acceptable salts and derivatives thereof. When using ursolic acid, uvaol, physiologically acceptable salts thereof or derivatives thereof in the antitumor agent according to the present invention, the origins of these substances are not restricted to particular ones and thus the substances may be those derived or isolated from natural resources, artificially synthesized ones and commercially available ones, with natural products being preferably used in the invention.

Ursolic acid is one of the ursane type triterpenes and a compound having a structure represented by the following chemical formula (3). It has been known that it possesses various effects such as an anti-inflammatory effect, an anti-arteriosclerotic effect, an anti-diabetic effect and an anti-lipemic effect (Jie Liu, Journal of Ethnopharmacology, 1995, 49:57-68). It has been known that this substance is widely distributed in the nature, for instance, fruits and leaves of apple, cherry and bearberry. In the antitumor agent of the present invention, the origins of ursolic acid, physiologically acceptable salts thereof or derivatives thereof are not restricted to specific ones at all and these substances may be

those derived or isolated from natural resources, artificially synthesized ones and commercially available ones, but preferred are those derived or isolated from natural resources such as apple, cherry and bearberry.

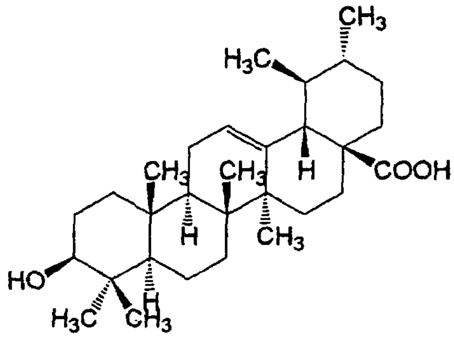

(3)

Regarding the ursolic acid, the foregoing is likewise true for the physiologically acceptable salts and derivatives thereof.

In this respect, examples of physiologically acceptable salts of ursolic acid include, but not limited to sodium ursolate, potassium ursolate, ammonium ursolate, dimethylammonium ursolate, calcium ursolate and magnesium ursolate.

Examples of derivatives of ursolic acid each derivatized at a single position include ursolic acid methyl ester, ursolic acid ethyl ester, ursolic acid n-propyl ester, ursolic acid isopropyl ester, ursolic acid n-butyl ester, ursolic acid trimethylsilyl ester, ursolic acid triethylsilyl ester, ursolic acid β -D-glucopyranosyl ester, ursolic acid β-D- galactopyranosyl ester, 3-O-acetyl-ursolic acid, 3-O-propionyl-ursolic acid, 3-O-butyryl- ursolic acid, 3-O-valeryl-ursolic acid, 3-O-capryl-ursolic acid, 3-O-lauryl-ursolic acid, 3-O-myristyl-ursolic acid, 3-O-palmityl-ursolic acid, 3-0-palmito-oleyl-ursolic acid, 3- O-stearoyl-ursolic acid, 3-O-oleyl-ursolic acid, 3-O-vaccenyl-ursolic acid, 3-O-linoleyl- ursolic acid, 3-O-linolenyl-ursolic acid, 3-O-arachidyl-ursolic acid, 3-O-arachidonyl- ursolic acid, 3-O-behenyl-ursolic acid, 3-O-methyl-ursolic acid, 3-O-ethyl-ursolic acid, 3-O-t-butyl-ursolic acid, 3-O-trimethylsilyl-ursolic acid, 3-O-triethylsilyl-ursolic acid, 3- O-benzyl-ursolic acid, 3-O- β -D-glucopyranosyl-ursolic acid, 3-O- β -D-galacto- pyranosyl-ursolic acid and 3-O-β -D-glucuronopyranosyl-ursolic acid.

Among these examples, preferably used herein include ursolic acid ethyl ester, ursolic acid β-D-glucopyranosyl ester, 3-O-acetyl-ursolic acid, 3-O-oleyl-ursolic acid, 3-O-linoleyl-ursolic acid, 3-O-linolenyl-ursolic acid and 3-O-β -D-glucopyranosyl-ursolic acid, with ursolic acid ethyl ester being particularly preferred in the present invention.

The foregoing are examples of the derivatives in which only one group is derivatized, but it is a matter of course that the derivatives may be derivatized at two or more possible and different positions or derivatized with at least two kinds of counterparts. In addition, only glycosides with monosaccharides are listed above, but it is likewise a matter of course that they may be glycosides with di or higher oligosaccharides comprising combinations of a variety of saccharides.

Uvaol is one of ursane type triterpenes, has a structure represented by the following chemical formula (4) and up to this time, it has been known as a substance possessing effects such as an anti-inflammatory effect (Planta. Med., 1995, Vol. 61, No.2, pp.182-185) and a glycerophosphate dehydrogenase-inhibitory effect (J.P. KOKAI No. Hei 9-67249). This substance is known to exist in the nature, for instance, olive, bearberry, sage, gum Senegal and cajeput tree. In the antitumor agent of the present invention, the origins of the uvaol or derivatives thereof are not restricted to any particular one and these substances may be those derived or isolated from natural resources, artificially synthesized ones and commercially available ones, with those derived from naturally-occurring materials such as olive, bearberry, sage, gum Senegal and cajeput tree being, for instance, preferably used herein. In particular, preferred are those derived from olive and more specifically, those derived from olive plants and/or products obtained in the olive oil-manufacturing processes.

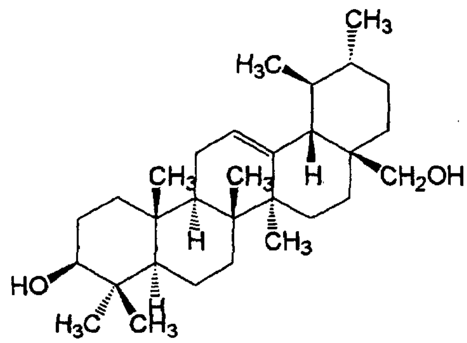

(4)

Regarding the uvaol, the foregoing is likewise true for the physiologically acceptable salts and derivatives thereof.

In this respect, examples of derivatives thereof each derivatized at a single position include, but not limited to 3-O-acetyl-uvaol, 3-O-propionyl-uvaol, 3-O-butyryl- uvaol, 3-O-valeryl-uvaol, 3-O-capryl-uvaol, 3-O-lauryl-uvaol, 3-O-myristyl-uvaol, 3-O- palmityl-uvaol, 3-O-palmito-oleyl-uvaol, 3-O-stearoyl-uvaol, 3-O-oleyl-uvaol, 3-O- vaccenyl-uvaol, 3-O-linoleyl-uvaol, 3-O-linolenyl-uvaol, 3-O-arachidyl-uvaol, 3-O- arachidonyl-uvaol, 3-O-behenyl-uvaol, 28-O-acetyl-uvaol, 28-O-propionyl-uvaol, 28-O- butyryl-uvaol, 28-O-valeryl-uvaol, 28-O-capryl-uvaol, 28-O-lauryl-uvaol, 28-O-myristyl- uvaol, 28-O-palmityl-uvaol, 28-O-palmito-oleyl-uvaol, 28-O-stearoyl-uvaol, 28-O-oleyl-uvaol, 28-O-vaccenyl-uvaol, 28-O-linoleyl-uvaol, 28-O-linolenyl-uvaol, 28-O-arachidyl- uvaol, 28-O-arachidonyl-uvaol, 28-O-behenyl-uvaol, 3-O-methyl-uvaol, 3-O-ethyl-uvaol, 3-O-t-butyl-uvaol, 3-O-trimethylsilyl-uvaol, 3-O-triethylsilyl-uvaol, 3-O-benzyl-uvaol, 28-O-methyl-uvaol, 28-O-ethyl-uvaol, 28-O-t-butyl-uvaol, 28-O-trimethylsilyl-uvaol, 28-O-triethylsilyl-uvaol, 28-O-benzyl-uvaol, 3-O- β -D-glucopyranosyl-uvaol, 3-O- β -D- galactopyranosyl-uvaol, 3-O- β -D-glucuronopyranosyl-uvaol, 28-O- β -D-glucopyranosyl- uvaol, 28-O- β -D-galactopyranosyl-uvaol and 28-O- β -D-glucuronopyranosyl-uvaol.

Among these uvaol derivatives, preferably used herein are 3-O-acetyl-uvaol, 28- O-acetyl-uvaol, 3-O-oleyl-uvaol, 3-O-linoleyl-uvaol, 3-O-linolenyl-uvaol, 28-O-oleyl- uvaol, 28-O-linoleyl-uvaol, 28-O-linolenyl-uvaol, 3-O- β -D-glucopyranosyl-uvaol and 28- O- β -D-glucopyranosyl-uvaol, with 3-O-acetyl-uvaol and 28- O-acetyl-uvaol being particularly preferred in the present invention.

The foregoing are examples of the derivatives in which only one group is derivatized, but it is a matter of course that the derivatives may be derivatized at two or more possible and different positions or derivatized with at least two kinds of counterparts. There may be listed, for instance, 3,28-O-diacetyl-uvaol. In addition, only glycosides with monosaccharides are listed above, but it is likewise a matter of course that they may be glycosides with di or higher oligosaccharides comprising combinations of a variety of saccharides.

Both of betulinic acid and betulin belong to the lupine type triterpene group and have been known to be present in a variety of plants. In addition, the foregoing is also true for the physiologically acceptable salts and derivatives thereof. When using betulinic acid, betulin, physiologically acceptable salts thereof or derivatives thereof in the antitumor agent of the present invention, the origins of these substances are not restricted to any particular one and these substances may be those derived or isolated from natural resources, artificially synthesized ones and commercially available ones, with naturally-occurring substances being preferably used in the present invention.

Betulinic acid is one of the lupine type triterpenes, has a structure represented by the following chemical formula (5) and up to this time, this compound has been known to have various effects such as carcinostatic, anti-inflammatory and wound healing- promotion effects (Japanese Examined Patent Publication (hereunder referred to as "J.P. KOKOKU") No. Hei 4-26623), an alcohol absorption-inhibitory effect (J.P. KOKAI No. Hei 7-53385) and a new hair growing-promotion effect (J.P. KOKAI No. Hei 9-157139). In the nature, this substance has been known to exist in, for instance, Ophelia japonica, clove, the rind of grape and olive in its free state; and in, for instance, Panax japonicus C.A. Meyer, carrot and sugar beet in the form of saponin. In the antitumor agent of the present invention, the origins of these betulinic acid, physiologically acceptable salts thereof or derivatives thereof are not restricted to particular ones and these substances may be those derived or isolated from natural resources, artificially synthesized ones and commercially available ones, with those derived from natural resources such as Ophelia japonica, clove, grape, olive, Panax japonicus C.A. Meyer, carrot and sugar beet being preferably used herein. In particular, preferred are those derived from olive and more specifically, those derived from olive plants and/or products obtained in the olive oil-manufacturing processes.

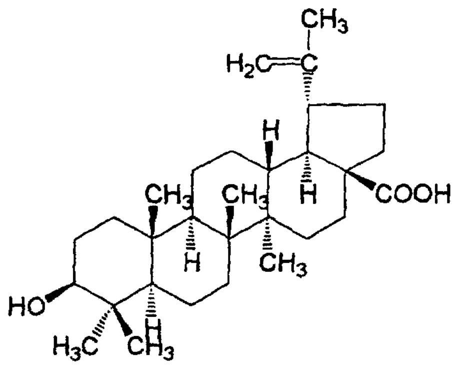

(5)

Regarding the betulinic acid, the foregoing is likewise true for the physiologically acceptable salts thereof and derivatives thereof.

In this respect, examples of physiologically acceptable salts of betulinic acid include, but not limited to, sodium betulinate, potassium betulinate, ammonium betulinate, dimethylammonium betulinate, calcium betulinate and magnesium betulinate, with sodium betulinate and potassium betulinate being preferably used herein.

Examples of betulinic acid derivatives, for instance, those derivatized only at a single position include betulinic acid methyl ester, betulinic acid ethyl ester, betulinic acid n-propyl ester, betulinic acid isopropyl ester, betulinic acid n-butyl ester, betulinic acid trimethylsilyl ester, betulinic acid triethylsilyl ester, betulinic acid- β -D- glucopyranosyl ester, betulinic acid- β-D-galactopyranosyl ester, 3-O-acetyl-betulinic acid, 3-O-propionyl-betulinic acid, 3-O-butyryl-betulinic acid, 3-O-valeryl-betulinic acid, 3-O-capryl-betulinic acid, 3-O-lauryl-betulinic acid, 3-O-myristyl-betulinic acid, 3-O-palmityl-betulinic acid, 3-O-palmito-oleyl-betulinic acid, 3-O-stearoyl-betulinic acid, 3- O-oleyl-betulinic acid, 3-0-vaccenyl-betulinic acid, 3-O-linoleyl-betulinic acid, 3-O- linolenyl-betulinic acid, 3-O-arachidyl-betulinic acid, 3-O-arachidonyl-betulinic acid, 3-O-behenyl-betulinic acid, 3-O-methyl-betulinic acid, 3-O-ethyl-betulinic acid, 3-O-t- butyl-betulinic acid, 3-O-trimethylsilyl-betulinic acid, 3-O-triethylsilyl-betulinic acid, 3- O-benzyl-betulinic acid, 3-O- β -D-glucopyranosyl-betulinic acid, 3-O- β -D-galacto-pyranosyl-betulinic acid and 3-O- β -D-glucuronopyranosyl-betulinic acid.

Among these betulinic acid derivatives, preferably used herein are betulinic acid ethyl ester, betulinic acid- β -D-glucopyranosyi ester, 3-O-acetyl-betulinic acid, 3-O- palmito-oleyl-betulinic acid, 3-O-linoleyl-betulinic acid, 3-O-linolenyl-betulinic acid and 3-O- β -D-glucopyranosyl-betulinic acid, with betulinic acid ethyl ester being particularly preferred in the present invention.

The foregoing are examples of the derivatives in which only one group is derivatized, but it is a matter of course that the derivatives may be derivatized at two or more possible and different positions or derivatized with at least two kinds of counterparts. In addition, only glycosides with monosaccharides are listed above, but it is likewise a matter of course that they may be glycosides with di or higher oligosaccharides comprising combinations of a variety of saccharides.

Betulin is one of the lupine type triterpenes, has a structure represented by the following chemical formula (6) and the compound has been known to possess a variety of effects such as a bio-protein denaturation-inhibitory action (J.P. KOKAI No. Hei 9-67253), a glycerophosphate dehydrogenase-inhibitory action (J.P. KOKAI No. Hei 9-67249) and a lipase-inhibitory action (J.P. KOKAI No. Hei 10-265328). This substance has been known to exist in the nature, for instance, in the bark of Japanese white birch. In the antitumor agent according to the present invention, the origins of betulin or derivatives thereof are not limited to any specific one and these substances may be those derived and isolated from natural resources, artificially synthesized ones and commercially available ones, with those derived from natural resources such as the bark of Japanese white birch being preferably used herein.

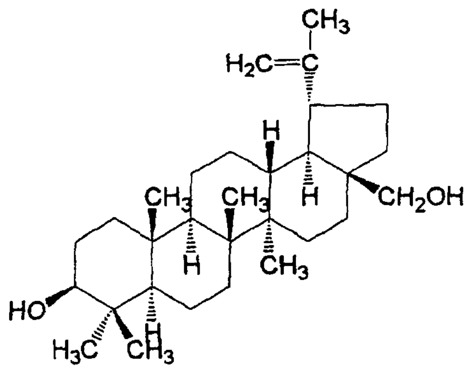

(6)

With respect to betulin, the foregoing is likewise true for the physiologically acceptable salts thereof and derivatives thereof.

In this respect, examples of derivatives of betulin, for instance, those derivatized at a single position include, but not limited to, 3-O-acetyl-betulin, 3-O-propionyl- betulin, 3-O-butyryl-betulin, 3-O-valeryl-betulin, 3-O-capryl-betulin, 3-O-lauryl-betulin 3-O- myristyl-betulin, 3-O-palmityl-betulin, 3-O-palmito-oleyl-betulin, 3-O-stearoyl-betulin, 3-O-oleyl-betulin, 3-O-vaccenyl-betulin, 3-O-linoleyl-betulin, 3-O-linolenyl-betulin, 3-O- arachidyl-betulin, 3-O-arachidonyl-betulin, 3-O-behenyl-betulin, 28-O-acetyl-betulin, 28-O-propionyl-betulin, 28-O-butyryl-betulin, 28-O-valeryl-betulin, 28-O-capryl-betulin, 28-O-lauryl-betulin, 28-O-myristyl-betulin, 28-O-palmityl-betulin, 28-O-palmito-oleyl- betulin, 28-O-stearoyl-betulin, 28-O-oleyl-betulin, 28-O-vaccenyl-betulin, 28-O-linoleyl- betulin, 28-O-linolenyl-betulin, 28-O-arachidyl-betulin, 28-O-arachidonyl-betulin, 28-O- behenyl-betulin, 3-O-methyl-betulin, 3-O-ethyl-betulin, 3-O-t-butyl-betulin, 3-O-tri- methylsilyl-betulin, 3-O-triethylsilyl-betulin, 3-O-benzyl-betulin, 28-O-methyl-betulin, 28-O-ethyl-betulin, 28-O-t-butyl-betulin, 28-O-trimethylsilyl-betulin, 28-O-triethylsilyl- betulin, 28-O-benzyl-betulin, 3-O- β -D-glucopyranosyl-betulin, 3-O- β -D-galacto- pyranosyl-betulin, 3-O- β -D-glucuronopyranosyl-betulin, 28-O- β -D-glucopyranosyl- betulin, 28-O- β -D-galacto- pyranosyl-betulin and 28-O- β -D-glucuronopyranosyl- betulin.

Among these betulin derivatives, preferably used herein are 3-O-acetyl-betulin, 28-O-acetyl-betulin, 3-O-oleyl-betulin, 3-O-linoleyl-betulin, 3-O-linolenyl-betulin, 28-O- oleyl-betulin, 28-O-linoleyl-betulin, 28-O-linolenyl-betulin, 3-O- β -D-glucopyranosyl- betulin and 28-O- β -D-glucopyranosyl-betulin, with 3-O-acetyl-betulin and 28-O-acetyl-betulin being particularly preferred.

The foregoing are examples of the derivatives in which only one group is derivatized, but it is a matter of course that the derivatives may be derivatized at two or more possible and different positions or derivatized with at least two kinds of counterparts. There may be listed, for instance, 3,28-O-diacetyl-betulin as a preferred example thereof. In addition, only glycosides with monosaccharides are listed above, but it is likewise a matter of course that they may be glycosides with di or higher oligosaccharides comprising combinations of a variety of saccharides.

These 5-membered ring-containing triterpenes as naturally occurring ones may be obtained by extracting from the respective plants such as those listed above. More specifically, they may be extracted from each plant body with water and/or organic solvents and they may be separated or isolated and purified by subjecting the resulting extract to a solvent-extraction method, a method, which makes use of the difference in solubility between the desired substance and impurities, a fractional precipitation method, a recrystallization method, an ion-exchange resin method and a liquid chromatography method, which may be used alone or in any appropriate combination, or may repeatedly be employed.

In particular, maslinic acid and/or physiologically acceptable salts thereof may be extracted from olive plants with water and/or an organic solvent and the desired substances may be separated or isolated and purified by subjecting the resulting extract to a solvent-extraction method, a method, which makes use of the difference in solubility between the desired substance and impurities, a fractional precipitation method, a recrystallization method, an ion-exchange resin method and a liquid chromatography method, which may be used alone or in any appropriate combination, or may repeatedly be employed.

Either of olive plants (Olea europaea L.) may be used in the present invention irrespective of their origins such as home-grown and Europe growth ones, or irrespective of whether they are edible ones or those for oil expression. The maslinic acid and/or physiologically acceptable salts thereof incorporated into theantitumor agent of the present invention may be obtained from principally seeds and fruits of olive plants as natural plants and further these substances may likewise be prepared from seed coats, leaves, stems and germs and/or buds of the olive plants. The substances may likewise suitably be obtained from dried, pulverized and/or defatted products of these raw materials.

Moreover, it is preferred to add water to the foregoing fruits of the olive plant or defatted products thereof, or

to subject the same to a humidifying treatment by, for instance, steaming the same, since these fruits of the olive plant or defatted products thereof get swollen to the desired degree and therefore, the extraction efficiency is significantly be improved.

In particular, it is preferred to use the defatted product of olive plants as a raw material, since maslinic acid and/or physiologically acceptable salts thereof are present therein in high concentrations and it is not necessary to remove oil components from the resulting maslinic acid and/or physiologically acceptable salts thereof.

The defatted product as a raw material may be oil expression residues derived from olive plants and produced during the edible oil-purifying processes, or residues discharged from extraction processes with a solvent such as hexane.

Moreover, the olive plants or the defatted product thereof may be extracted with at least one solvent selected from, for instance, a hydrocarbon solvent such as pentane, hexane and heptane, a lower fatty acid alkyl ester such as acetic acid ethyl ester and known non-aqueous organic solvents such as diethyl ether to thus remove the lipid components included therein and this washing (or extraction) step is if desired repeated to give a defatted product suitably used in the present invention.

Maslinic acid and/or physiologically acceptable salts thereof to be incorporated into the antitumor agent of the present invention can thus be produced by extracting the foregoing olive plants with water and/or an organic solvent.

Such an organic solvent used for preparing maslinic acid and/or physiologically acceptable salts thereof from olive plants may be either hydrophilic organic solvents or hydrophobic organic solvents. Specific examples thereof are known organic solvents, for instance, alcohols such as methyl alcohol, ethyl alcohol, glycerin, propylene glycol and 1,3-butylene glycol; acetone, tetrahydrofuran, acetonitrile, 1,4-dioxane, pyridine, dimethylsulfoxide, N,N-dimethylformamide and acetic acid as the hydrophilic organic solvents and known organic solvents such as hexane, cyclohexane, carbon tetrachloride, chloroform, dichloromethane, 1,2-dichloroethane, diethyl ether, ethyl acetate, benzene and toluene as the hydrophobic organic solvents. Moreover, these organic solvents may be used alone or in any combination of at least two of them.

From the industrial standpoint, for instance, from the viewpoint of the ability of penetrating into the plant's tissues and the extraction efficiency, it is preferred to use hydrophilic organic solvents and moisture-containing hydrophilic organic solvents. Specific examples thereof are alcohols such as methyl alcohol, ethyl alcohol, glycerin, propylene glycol and 1,3-butylene glycol; other organic solvents such as acetone, tetrahydrofuran and acetonitrile; and these organic solvents containing water. Maslinic acid and/or physiologically acceptable salts thereof to be incorporated into the antitumor agent of the present invention can thus be produced by extracting the foregoing olive plants with a solvent comprising at least one member selected from the foregoing organic solvents.

The conditions for the extraction are not particularly restricted, but the extraction temperature, for instance, ranges from 5 to 95°C, preferably 10 to 90°C and more preferably 15 to 85°C and further the extraction may likewise suitably be conducted at ordinary temperature. There is such a tendency that the higher the extraction temperature, the higher the extraction efficiency. The extraction procedures may favorably be conducted at ordinary pressure, under pressure or reduced pressure established by, for instance, aspiration. Moreover, it is also possible to conduct the extraction procedures in accordance with the shaking or oscillating extraction or using an extractor equipped with a stirring machine for the improvement of the extraction efficiency. The extraction time may vary depending on other extraction conditions, but in general ranges from several minutes to several hours. The longer the extraction period of time, the higher the extraction efficiency, but the extraction time may appropriately be determined while taking into consideration various production conditions such as production facilities and production yield.

In the foregoing extraction procedures, the solvent may be used in an amount ranging from 1 to 100 times (mass/mass; the same definition will apply to the following) and preferably I to 20 times the amount of the raw material.

In this respect, it is particularly preferred to conduct the extraction using either of water, moisture-containing lower alcohols and anhydrous lower alcohols if taking into consideration the safety thereof to human bodies or the like.

Moreover, it is preferred to conduct the extraction using a water-containing lower alcohol having a lower alcohol content of not less than 10% by mass if taking into consideration the yield of the resulting maslinic acid and/or physiologically acceptable salts thereof and the intensity of theantitumor effect thereof. The water- containing alcohol used herein more preferably has a lower alcohol content ranging from 10% by mass to 95% by mass and most preferably the water-containing alcohol used herein has a lower alcohol content adjusted to the range of from 30% by mass to 95% by mass.

In this connection, the alcohols usable in the present invention may be, for instance, primary alcohols such as methyl alcohol, ethyl alcohol, 1-propanol and 1-butanol; secondary alcohols such as 2-propanol and 2-butanol; tertiary alcohols such as 2-methyl-2-propanol; and liquid polyhydric alcohols such as ethylene glycol, propylene glycol and 1,3-butylene glycol. These solvents may be used alone or in any combination of at least two of them.

The term "lower alcohol" herein used means known alcohols having 1 to 4 carbon atoms such as the primary, secondary, tertiary and liquid polyhydric alcohols listed above and these solvents may be used alone or in any combination of at least two thereof.

The maslinic acid and/or physiologically acceptable salts thereof used in the present invention can be obtained by removing the solvents and moisture from the crude extract thus obtained.

The solvents and the moisture can be removed from such a crude extract according to a known method such as distillation under reduced pressure (or vacuum distillation), drying in vacuo or under reduced pressure, freeze-drying (or lyophilization) and spray drying.

The condition of the final product is not limited to any particular one and the product may be used without removing the solvent and moisture.

In the present invention, the extract derived from the defatted product is preferred since it is free of any oil-soluble components such as triglycerides, sterols and tocopherol and therefore, it is not needed to remove such impurities or to purify the extracted product. Moreover, the defatted product comprises residues after the oil expression and in other words, compressed lees and extracted lees remaining after the oil expression of olive oil can be used in the present invention. Therefore, the method is quite excellent one, which allows the effective use of olive and it is also considered to be excellent from the viewpoint of the production cost since the method uses materials generally disposed or used as feeds.

Moreover, it is preferred to subject, to concentration treatments or the like, the extract containing maslinic acid and/or physiologically acceptable salts thereof to be incorporated into the antitumor agent of the present invention in order to further improve theantitumor effect of the maslinic acid and/or physiologically acceptable salts thereof extracted from olive plants.

The conditions for concentrating the crude extract are not restricted to specific ones, but examples thereof include those making use of the difference in solubility in water between components present in the extract. Maslinic acid and/or physiologically acceptable salts thereof incorporated into theantitumor agent of the present invention are compounds having relatively low polarity and hardly soluble in water. The crude extract derived from olive plants is divided into components hardly soluble in water and/or components insoluble in water or hardly water-soluble or water-insoluble components and components easily soluble in water, while making use of the foregoing characteristic properties of maslinic acid and/or physiologically acceptable salts thereof, to thus substantially concentrate the crude extract. The hardly water-soluble or water-insoluble components included in the crude extract derived from olive plants are substantially improved in the antitumor effect as compared with that observed for the whole crude extract derived from olive plants and thus it can be confirmed that in the resulting concentrate, maslinic acid and/or physiologically acceptable salts thereof are substantially concentrated.

The hardly water-soluble or water-insoluble components can easily be obtained by adding the crude extract from olive plants to water with stirring and then collecting the precipitated portion through, for instance, filtration.

In addition, maslinic acid and/or physiologically acceptable salts thereof incorporated into theantitumor agent of the present invention can, if needed, be concentrated by a liquid-liquid partition technique using a combination of usual solvents. It would be difficult to unconditionally determine such combination of solvents, but examples thereof include combinations of water and hydrophobic organic solvents. In this respect, specific examples of such hydrophobic organic solvents are known organic solvents such as hexane, carbon tetrachloride, chloroform, dichloromethane, 1,2-dichloroethane, diethyl ether, ethyl acetate, n-butanol, benzene and toluene.

Maslinic acid and/or physiologically acceptable salts thereof are hardly soluble in water and therefore, the resulting hydrophobic organic solvent phase is fractionated to thus remove the undesirable water-soluble components. Thereafter, the solvent is removed to thus easily concentrate maslinic acid and/or physiologically acceptable salts thereof.

Moreover, maslinic acid and/or physiologically acceptable salts thereof to be incorporated into the antitumor agent of the present invention are preferably prepared by additionally fractionating and/or purifying the foregoing extract and/or the concentrate. This treatment permits the concentration of maslinic acid and/or physiologically acceptable salts thereof to a concentration factor higher than that achieved by the foregoing treatment and the isolation of the intended components.

The fractionation and/or purification treatments are, for instance, quite advantageous in that they permit the considerable improvement of theantitumor effect of the resulting product and that they likewise permit the removal of the impurities. In other words, when the foregoing extract and/or the concentrate are subjected to these fractionation and/or purification treatments, maslinic acid and/or physiologically acceptable salts thereof can be recovered as white crystals and therefore, these treatments are preferred since the treatments have such an advantage that the product may be incorporated into theantitumor agent without entraining undesirable coloring of the agent.

The method for fractionation and/or purification usable herein cannot be unconditionally defined, but examples thereof usable herein are those which make use of, for instance, a recrystallization technique, a fractional precipitation technique and a chromatography technique. The method which makes use of the chromatography technique, in particular, the liquid chromatography technique is preferred since the method permits the fractionation and/or purification of maslinic acid and/or physiologically acceptable salts thereof to be incorporated into the antitumor agent of the present invention in a high yield without causing any decomposition of the foregoing components. Specific examples of liquid

chromatography techniques are normal phase liquid chromatography, reversed phase liquid chromatography, thin layer chromatography, paper chromatography and high performance liquid chromatography (HPLC) techniques. Either of these techniques can be used in the fractionation and/or purification of maslinic acid and/or physiologically acceptable salts thereof. In particular, normal phase liquid chromatography, reversed phase liquid chromatography and high performance liquid chromatography (HPLC) techniques are preferred in the present invention, while taking into consideration, for instance, the resolution, throughput rate and number of steps required to use.

In this respect, the term "normal phase liquid chromatography" means, for instance, the following method. More specifically, the method comprises the steps of, for instance, preparing a column whose stationary phase comprises silica gel and whose mobile phase comprises, for instance, a hexane-ethyl acetate mixed solvent or a chloroform-methanol mixed solvent; supplying or loading the crude extract derived from olive plants or a concentrate thereof on the column at a loading rate ranging from 0.1 to 5% (wt (mass)/v (volume)); and then eluting a desired fraction according to a continuous elution method using a single mobile phase or a stepwise elution method in which the polarity of the solvent used is successively increased.

The reversed phase liquid chromatography technique means, for instance, the following method. More specifically, the method comprises the steps of preparing a column whose stationary phase comprises octadecylsilane-linked silica (ODS) and whose mobile phase comprises, for instance, a water-methanol mixed liquid, a water-acetonitrile mixed liquid or a water-acetone mixed liquid; supplying or loading the crude extract derived from olive plants or a concentrate thereof on the column at a loading rate ranging from 0.1 to 5% (wt (mass)/v (volume)); and then eluting a desired fraction according to a continuous elution method using a single mobile phase or a stepwise elution method in which the polarity of the solvent used is successively increased.

The high performance liquid chromatography (HPLC) is identical, in principle, to those of the foregoing normal phase liquid chromatography or reversed phase liquid chromatography and this is a chromatography technique for fractionation and/or purification at a high speed and a high resolution.

The foregoing methods can preferably be used alone or in any combination of at least two of them and thus maslinic acid and/or physiologically acceptable salts thereof can be concentrated to a substantial degree and the resulting product is substantially free of impurities.

Further, the purity of maslinic acid and/or physiologically acceptable salts thereof can be adjusted and the intensity of the antitumor effect and the characteristic properties of the product can if necessary be arbitrarily designed using the foregoing methods, which may be used alone or in any combination of at least two of them.

Regarding the foregoing concentration treatment, it may preferably be repeated and further different concentration treatments may be used in combination. Similarly, with respect to the fractionation-purification treatment, the treatment may preferably be repeated and further different fractionation-purification treatments may be used in combination. Moreover, it is possible to carry out the fractionation-purification treatment after the completion of the concentration treatment; to carry out the concentration treatment after the completion of the fractionation-purification treatment; or to carry out the fractionation-purification treatment after the completion of the concentration treatment and then again carry out an additional concentration treatment. Combinations other than those described above may likewise be used in the present invention.

Maslinic acid and/or physiologically acceptable salts thereof can suitably be obtained by variously combining, for instance, the foregoing extraction treatment, concentration treatment, fractionation and/or purification treatments. Such combination is not restricted to specific ones, but specific examples of such a series of treatments are as follows:

For instance, after the olive plants are extracted with water and/or a hydrophilic organic solvent, a part or the whole of the hydrophilic organic solvent is removed from the resulting extract and the water-insoluble fraction precipitated in the aqueous phase is recovered to thus concentrate the extract. The precipitated water-insoluble fraction can be recovered by a means such as filtration and centrifugation, but the resulting aqueous solution may if necessary be subjected to additional treatments such as addition of water and stirring for the improvement of the rate of recovery thereof. In addition, an extract in dry condition obtained by removing water and/or the hydrophilic organic solvent from the extract derived from olive plants may likewise be subjected to additional treatments such as addition of water and stirring similar to those used above, followed by the recovery of the resulting insolubles in water through filtration to thus concentrate the dried extract. This concentration method is preferred since it is a treatment in an aqueous system, it is accordingly excellent in the safety as compared with the concentration using a solvent and a wide variety of machinery and tools may be used. Moreover, this method is also preferred since the dry extract as a starting material is almost free of any oil component and it is excellent in the concentration and/or purification efficiency.

Highly purified maslinic acid and/or physiologically acceptable salts thereof may favorably be obtained by fractionating and/or purifying these concentrates in accordance with normal phase and/or reversed phase chromatography and/or recrystallization.

Alternatively, the extract derived from olive plants may likewise be concentrated by the liquid-liquid partition technique using a water-hydrophobic organic solvent system. For instance, the hydrophilic organic solvent is removed from the extract, water is if necessary added to the remaining aqueous solution and then a hydrophobic organic solvent

is added to the resulting aqueous phase. In addition, the extract in a dried condition may likewise be concentrated by the liquid-liquid partition technique using a water-hydrophobic organic solvent system. For instance, water is added to the dry extract like the method described above and then a hydrophobic organic solvent is added to the resulting aqueous phase. High purity maslinic acid and/or physiologically acceptable salts thereof may favorably be obtained by fractionating and/or purifying these concentrates in accordance with normal phase and/or reversed phase chromatography and/or recrystallization.

If an antitumor agent, a tumor cell-proliferation-inhibitory agent, a tumor cell-killing agent or a tumor cell-metastasis-inhibitory agent is prepared using an isolated product derived from natural raw material and highly purified, the effect of each agent can be improved, any influence of contaminants on each agent may be eliminated and colorless to pale colored and/or odorless to almost odorless agents can be prepared. For this reason, the highly purified isolated product is preferably used since any carrier and sweetening agent may be used without any limitation in the preparation of these agents.

In addition, maslinic acid or the like as the subject of the present invention is contained in natural raw materials, but the product isolated from these raw materials may be derivatized into salts and derivatives. As a result, the solubility of the product in water or oil may be improved and therefore, the product can widely be designed in the characteristic properties such as the effects, quality and/or handling ability although the natural products per se such as olive oil are limited in the applications as pharmaceutical agents. For instance, when pharmaceutical preparations are prepared by the incorporation of oils and fats, the natural raw material as such is limited in the content of, for instance, maslinic acid and the amounts and kinds of effective components capable of being combined together. When using a product isolated from natural raw materials and salts or derivatives thereof, however, one can enjoy a variety of advantages such that a desired amount of an effective component such as maslinic acid can be ensured and that additives such as excipients and auxiliary agents may appropriately be selected depending on the dosage forms. Moreover, the effect of each effective component can be improved as compared with those expected when the natural raw material is taken in without any pre-treatment.

In addition, the purity of, for instance, maslinic acid used in the present invention is preferably improved since this would permit the reduction of side effects due to unidentified substances possibly present in the natural raw materials.

Moreover, the total content of maslinic acid and physiologically acceptable salts thereof in the mixture of maslinic acid and physiologically acceptable salts derived from olive plants and/or the product obtained in the olive oil-manufacturing processes is preferably not less than 95% and more preferably 95% to 99.99%. This content can be determined according to, for instance, the gas chromatography technique.

The antitumor agent of the present invention comprises maslinic acid and/or physiologically acceptable salts thereof, but theantitumor agent can be prepared, as well, by the incorporation of the foregoing extract and the concentrate. Moreover, the degrees of, for instance, concentration and purification may be controlled to adjust, for instance, the concentrations of maslinic acid and/or physiologically acceptable salts thereof and thus, the product whose degrees of concentration and purification are controlled may suitably be incorporated into antitumor agents. More specifically, when a stronger effect is required, the product is concentrated, while in case where it is sufficient that the product has a rather weak effect, a diluted product may be used. Thus, the concentration of the effective components of the resulting product can arbitrarily be selected depending on the applications.

Moreover, otherantitumor agents may be used in combination with that of the present invention and accordingly, the antitumor effect of the resulting agent can be designed in detail and it would be expected to considerably reinforce theantitumor effect through the synergistic effect with otherantitumor agents. More specifically, the antitumor effect of an intendedantitumor agent can be designed by appropriately adjusting the intensity of theantitumor effect and the mechanism of action. The intensity of theantitumor effect can be controlled by concentrating the product containing the effective components when a stronger effect is required, and by diluting the product when a rather weak effect is required or sufficient. The intensity of the antitumor effect can thus be controlled depending on each specific application. Alternatively, the antitumor effect of an agent can likewise be controlled by combining maslinic acid or the like of the invention with other antitumor agents other than those of the present invention. The mechanisms of the antitumor effect may be, for instance, the inhibition of tumor cell-proliferation, the killing of tumor cells and the inhibition of tumor cell-metastasis. Such actions can be adjusted by combining maslinic acid or the like with antitumor agents other than those of the present invention.

In the antitumor agent of the present invention, olive oil is preferably used as an oily component to thus obtain more favorable effects such as an antitumor effect since the olive oil comprises, for instance, maslinic acid.

Moreover, when maslinic acid and/or physiologically acceptable salts thereof are extracted from olive plants, oleanolic acid and/or physiologically acceptable salts thereof are also extracted simultaneous with the maslinic acid or the like. The oleanolic acid and/or physiologically acceptable salts thereof have a carcinogenic promoter-inhibitory activity and are excellent in the compatibility with maslinic acid. Therefore, the mixture of these components can directly be incorporated into theantitumor agent of the present invention. The use of such a mixture is preferred since the

antitumor effect of maslinic acid and/or physiologically acceptable salts thereof may be improved due to the synergistic effect with the oleanolic acid or the like. When extracting, isolating and purifying maslinic acid and/or physiologically acceptable salts thereof from olive plants, appropriate adjustment of the conditions therefor would permit the extraction of oleanolic acid and/or physiologically acceptable salts thereof as a mixture of the former. It is also possible to separately extract maslinic acid and/or physiologically acceptable salts thereof, and oleanolic acid and/or physiologically acceptable salts thereof, from olive plants and then admix these components. Atternahvely, it is also possible to admix maslinic acid and/or physiologically acceptable salts thereof with oleanolic acid and/or physiologically acceptable salts thereof, which are isolated from different raw materials respectively.

Maslinic acid, erythrodiol, ursolic acid, uvaol, betulinic acid, betulin and physiologically acceptable salts thereof or derivatives thereof capable of being incorporated into the antitumor agent of the present invention haveantitumor effects. More specifically, the effects may, for instance, be a tumor cell-proliferation-inhibitory action, a tumor cell-killing action and a tumor cell-metastasis-inhibitory effect.

The tumor cell-proliferation-inhibitory action is to inhibit any further proliferation or growth of tumor cells, in particular, cancer cells already developed in a living body so that these cells adversely affect the living body no longer. This effect clinically permits the interception of the progress of cancer and the daily uptake of the agent shows a prophylactic effect or may considerably contribute to the inhibition of the progress of tumor cells in an invisible level.

The tumor cell-killing effect means an effect such that the agent makes it impossible for tumor cells, in particular, cancer cells already developed in a living body to maintain any action or activity of the cells. This effect clinically permits the restoration of a patient suffering from a cancer to his normal body and the daily uptake of the agent shows a prophylactic effect or may considerably contribute to the extinction of tumor cells developed in an invisible level.

The tumor cell-metastasis-inhibitory effect is an effect such that in the process wherein tumor cells, in particular, cancer cells developed in a living body are transported to other sites through, for instance, the blood stream and undergo proliferation, the agent permits the extinction of cancer cells when the cells are present in the blood stream or permits the cell-proliferation-inhibition or the extinction of the cells at an instance when they arrive at other sites. This effect clinically permits the prevention of the cancer from spreading throughout the living body and the daily uptake of the agent shows a prophylactic effect or may considerably contribute to the inhibition or control of the tumor cell-metastasis caused in an invisible level.

The tumor cell-proliferation-inhibitory effect and the tumor cell-killing or extinction effect can be determined according to the following method using B-16 melanoma cells.

More specifically, B-16 melanoma cells are inoculated in each well of a 6-well plate in a desired amount, followed by allowing the plate to stand at 37°C and 5% carbon dioxide concentration to thus cultivate the cells, adding a sample solution to be tested (maslinic acid, erythrodiol, ursolic acid, uvaol, betulinic acid, betulin and physiologically acceptable salts thereof or derivatives thereof) to each well in an amount sufficient for achieving a desired concentration, on the next day or after 5 days from the initiation of the cultivation, determination of viable cell count on the $6^{th}$ day after the initiation of the cultivation and calculation of the cell growth rate based on the viable cell count to thus evaluate the tumor cell-proliferation-inhibitory effect and the tumor cell-killing effect. The results thus obtained are compared with the cell growth rate observed when any test sample is not added (control).

Maslinic acid, erythrodiol, ursolic acid, uvaol, betulinic acid, betulin and physiologically acceptable salts thereof or derivatives thereof can inhibit the proliferation of the B-16 melanoma cells or extinguish the B-16 melanoma cells, in a concentration-dependent manner even when they are added at a quite low concentration. In other words, maslinic acid, erythrodiol, ursolic acid, uvaol, betulinic acid, betulin and physiologically acceptable salts thereof or derivatives thereof possess quite strong tumor cell-proliferation-inhibitory effect and the tumor cell-killing effect. Oleanolic acid known as an inhibitor of carcinogenic promoter cannot inhibit the proliferation of the B-16 melanoma cells at all, while maslinic acid, erythrodiol, ursolic acid, uvaol, betulinic acid, betulin and physiologically acceptable salts thereof or derivatives thereof used in the present invention show quite excellent tumor cell-proliferation-inhibitory effect and the tumor cell-killing effect.

Moreover, these substances are effective even at a low concentration, the amount of these substances required for achieving a desired or expected tumor cell-proliferation-inhibitory effect and the tumor cell-killing effect is relatively small and therefore, they would show the foregoing effects without accompanying a high risk of possible side effects. These substances are effective in a concentration-dependent manner and accordingly, the amount thereof to be added can arbitrarily be controlled in response to the purpose of use and the intensity of the effect required.

The tumor cell-metastasis-inhibitory effect can be evaluated according to, for instance, the malignant melanoma metastasis-inhibitory test. More specifically, a suspension of B16 melanoma cells prepared in advance is intravenously injected into whister female rats, cotton seed oil in which each test substance is dissolved in a predetermined concentration is intraperitoneally administered through injection or orally administered using a sonde, every second day starting from $2^{nd}$ day after the intravenous injection of the suspension. In this connection, only cotton seed oil is administered to the control group of animals. The lungs are extracted from these animals on the $15^{th}$ day after the injection of the B16 melanoma cells, the lungs are examined to determine the number of cancer lesions thus metastasized to thus calculate the rate of metastasis-inhibition. The tumor cell-metastasis-inhibitory effect of each test sample is evaluated on the basis of the resulting rate of metastasis-inhibition.

Regarding the evaluation of the tumor cell-metastasis-inhibitory effect, there is not observed any significant difference in this effect between the group to which oleanolic acid as a known inhibitor for carcinogenic promoter is administered and the control group (no administrated effective component) or oleanolic acid does not possess any tumor cell-metastasis-inhibitory effect. On the other hand, there are observed significant differences in this effect between the groups of animals to which maslinic acid, erythrodiol, ursolic acid, uvaol, betulinic acid, betulin and physiologically acceptable salts thereof or derivatives thereof are administered and the control group (no administrated effective component) and this clearly indicates that these substances can inhibit any metastasis of malignant melanoma cells. In other words, maslinic acid, erythrodiol, ursolic acid, uvaol, betulinic acid, betulin and physiologically acceptable salts thereof or derivatives thereof possess quite strong tumor cell-metastasis-inhibitory effects.

These substances can show the desired effects even at a relatively small dose. Therefore, only a small amount of these substances is required for ensuring the achievement of a desired tumor cell-metastasis-inhibitory effect and they would show the foregoing effect without accompanying a high risk of possible side effects. These substances are effective in a concentration-dependent manner and accordingly, the amount thereof to be added can arbitrarily be controlled in response to the purpose of use and the intensity of the effect required.

The present invention also relates to a method of using one or at least two members selected from the group consisting of maslinic acid, erythrodiol, ursolic acid, uvaol, betulinic acid, betulin and physiologically acceptable salts thereof or derivatives thereof as an antitumor agent for the purposes of, in particular, the inhibition of tumor cell-proliferation, the extinction of tumor cells and/or the inhibition of tumor cell-metastasis. It is a matter of course that theantitumor agent of the present invention can specifically and separately be used as a tumor cell-proliferation-inhibitory agent, a tumor cell-killing agent or a tumor cell-metastasis-inhibitory agent.

The antitumor agent of the present invention is used as a prophylactic agent or a therapeutic agent having tumor cell-proliferation-inhibitory, tumor cell-killing and tumor cell-metastasis-inhibitory effects.

When theantitumor agent of the present invention is used as a prophylactic agent, it may be used for the purpose of the inhibition of any tumor-formation and for the purpose of inhibiting the tumor cell-proliferation and extinguishing the tumor cells immediately after the development of tumor cells. When theantitumor agent is used as a prophylactic agent, the agent effectively shows the effect thereof by, for instance, regularly taking a constant amount of the agent in the usual life. Thus, such prophylactic use of the antitumor agent of the invention would permit the inhibition of any development of tumor and even when there is development of tumor cells in quite initial stage, which does not show any subjective symptom and which cannot be detected even in the medical examination or diagnosis in a medical institution, the agent can control and/or extinguish the proliferation of the developed tumor cells and the agent possesses quite preferred functions as a prophylactic agent. When the antitumor agent of the present invention is used as a therapeutic agent, the agent can be used for the purpose of suppressing any proliferation of tumor cells and/or killing the same and inhibiting any metastasis of the tumor cells and, in other words, the agent can be used for the purpose of interrupting any progress of the tumor and extinguishing the same. One or at least two members selected from the group consisting of maslinic acid, erythrodiol, ursolic acid, uvaol, betulinic acid, betulin and physiologically acceptable salts thereof or derivatives thereof, which are incorporated into theantitumor agent of the present invention sufficiently show their effects even at a small dose and therefore, these substances may provide therapeutic agents, which possess a sufficient tumor cell-proliferation-inhibitory action and which have almost no side effect and high safety. Moreover, when one or at least two members selected from the group consisting of maslinic acid, erythrodiol, ursolic acid, uvaol, betulinic acid, betulin and physiologically acceptable salts thereof or derivatives thereof are used in an amount of not less than two times that required for showing the tumor cell-proliferation-inhibitory effect, the resulting agent would be expected as a therapeutic agent for extinguishing tumor cells. Further, any metastasis of tumor cells can be inhibited due to the foregoing tumor cell-proliferation-inhibitory action and tumor cell-killing effect and accordingly, the agent of the present invention is also effective as a therapeutic agent having a tumor cell-metastasis-inhibitory effect.

The antitumor agent of the present invention can orally or parenterally and stably administered to human and animals as, for instance, a drug or a quasi-drug. In this respect, examples of parenteral administration include intravenous injection, intra-arterial injection, intramuscular injection, subcutaneous injection, intracutaneous injection, intraperitoneal injection, intra-spinal injection, peridural injection, percutaneous administration, perpulmonary administration, pernasal administration, perintestinal administration, administration through oral cavity and permucosal administration and examples of dosage forms used in such perenteral administration routes include injections, suppositories (such as rectal suppositories, urethral suppositories and vaginal suppositories), liquids for external use (such as injections, gargles, mouth washes, fomentations, inhalants, sprays, aerosols, enema, paints, cleaning agents, disinfectants, nasal drops and ear drops), cataplasms, percutaneous absorption tapes, external preparations for the skin, ointments (such as pastes, liniments and lotions). Among these, preferred are injections, liquids for external use and external preparations for the skin. In addition, examples of pharmaceutical preparations for oral administration include tablets

for internal use (such as uncoated tablets, sugar-coated tablets, coating tablets, enteric coated tablets and chewable tablets), tablets administered to oral cavity (such as buccal preparations, sublingual tablets, troches and adhesive tablets), powders, capsules (such as hard capsules and soft capsules), granules (such as coated granules, pills, troches, liquids preparations or pharmaceutically acceptable sustained release pharmaceutical preparations). Specific examples of liquid preparations capable of being orally administered are solutions for internal use, shake mixtures, suspensions, emulsions, syrups, dry syrups, elixirs, infusion and decoction and limonades. Among these, preferred are tablets for internal use, powders, capsules and granules.

These pharmaceutical preparations are administered, as pharmaceutical compositions, in combination with, for instance, pharmaceutically acceptable carriers and/or diluents in accordance with the known pharmaceutical preparation method.

Examples of carriers and excipients used in these pharmaceutical preparations are lactose, glucose, sucrose, mannitol, potato starch, corn starch, calcium carbonate, calcium phosphate, calcium sulfate, crystalline cellulose, powdered glycyrrhiza and powdered gentian. The content of these additives in the antitumor agent, tumor cell-proliferation-inhibitory agent, tumor cell-killing agent or tumor cell-metastasis-inhibitory agent according to the present invention is not restricted to specific one, but it preferably ranges from 0 to 95% by mass.

In these pharmaceutical preparations, binders are used and examples thereof are starch, tragacanth gum, gelatin, syrups, polyvinyl alcohol, polyvinyl ether, polyvinyl pyrrolidone, hydroxypropyl cellulose, methyl cellulose, ethyl cellulose and carboxy- methyl cellulose. The content of these binders in the antitumor agent, tumor cell-proliferation-inhibitory agent, tumor cell-killing agent or tumor cell-metastasis-inhibitory agent according to the present invention is not restricted to specific one, but it preferably ranges from 0 to 95% by mass.

These pharmaceutical preparations may comprise a disintegrator and examples thereof are starch, agar, powdered gelatin, sodium carboxymethyl cellulose, calcium carboxymethyl cellulose, crystalline cellulose, calcium carbonate, sodium hydrogen carbonate and sodium alginate. The content of the disintegrator in the antitumor agent, tumor cell-proliferation-inhibitory agent, tumor cell-killing agent or tumor cell-metastasis-inhibitory agent according to the present invention is not restricted to specific one, but it preferably ranges from 0 to 95% by mass.

These pharmaceutical preparations may likewise comprise a lubricant and examples thereof usable herein are magnesium stearate, talc, hydrogenated vegetable oils and macrogol. The content of the lubricant in the antitumor agent, tumor cell-proliferation-inhibitory agent, tumor cell-killing agent or tumor cell-metastasis-inhibitory agent according to the present invention is not restricted to specific one, but it preferably ranges from 0 to 95% by mass.

These pharmaceutical preparations may likewise comprise a pigment and such a pigment may be, for instance, pharmaceutically acceptable one.

In addition, when preparing an injectable solution, an auxiliary agent for solubilization such as a pH-adjusting agent, a buffering agent, a stabilizer, a solubilizing agent, anhydrous ethanol, propylene glycol and liquid polyethylene glycol; and/or a surfactant such as polyoxyethylene hardened castor oil, Polysorbate 80 and 20 are if necessary added to the foregoing ingredients and then each desired injection is prepared according to the usual method.

When preparing a tablet or a granule, the tablet or granule may if required, be coated with sucrose, gelatin, hydroxypropyl cellulose, purified shellac, gelatin, glycerin, sorbitol, ethyl cellulose, hydroxypropyl cellulose, hydroxypropyl methyl cellulose, polyvinyl pyrrolidone, cellulose acetate phthalate, hydroxypropyl methyl cellulose phthalate, methyl methacrylate, methacrylic acid polymers or it may be coated with at least two layers of these materials. Moreover, it may further be a capsule made of a substance such as ethyl cellulose or gelatin.

Preparations for external use may be, for instance, solid, semi-solid, semi-solid-like or liquid-like pharmaceutical preparations for percutaneous administration or permucosal administration such as administration through oral cavity or pernasal administration.

Examples of liquid pharmaceutical preparations include pharmaceutically acceptable emulsions such as latex or milky lotions and lotions, tinctures for external use and liquid preparations for permucosal administration. The pharmaceutical preparation may comprise, for instance, ethanol, oil components, emulsifying agents as commonly used diluents.

Examples of semi-solid pharmaceutical preparations include ointments such as oil ointments and hydrophilic ointments. These semi-solid pharmaceutical preparations comprise, for instance, water, vaseline, polyethylene glycol, oil components and/or surfactants, as commonly used bases or carriers.

Examples of semi-solid or solid pharmaceutical preparations are adhesive agents for percutaneous administration or permucosal administration (for instance, the administration through oral cavity or pernasal administration) such as plaster preparations (such as rubber plaster and plaster), film preparations, tape-like preparations or cataplasm. This pharmaceutical preparation may comprise, for instance, rubber polymers such as natural rubber, synthetic rubber such as butadiene rubber, SBR and SIS; gelatin, sludge-forming agents such as kaolin and zinc oxide; hydrophilic polymers such as sodium carboxymethyl cellulose and sodium polyacrylate; tackifiers such as acrylic resins and liquid paraffin; water, other oil components and/or surfactants, as commonly used bases or carriers.

These pharmaceutical preparations may further comprise an auxiliary agent such as a stabilizer, an auxiliary

agent for solubilization, a percutaneous absorption promoter; and other additives such as an aromatic and a preservative.

The antitumor agent, tumor cell-proliferation-inhibitory agent, tumor cell-killing agent or tumor cell-metastasis-inhibitory agent according to the present invention may directly be applied to external and/or internal tumor lesions of human bodies to thus enjoy the effects of these agents or may be administered through the oral route or through injection to likewise enjoy the effects of these agents: The content of maslinic acid, erythrodiol, ursolic acid, uvaol, betulinic acid, betulin and physiologically acceptable salts thereof or derivatives thereof in the antitumor agent, tumor cell-proliferation-inhibitory agent, tumor cell-killing agent or tumor cell-metastasis-inhibitory agent according to the present invention may vary depending on variety of factors such as methods for the application, intake and administration and the term thereof and the dosage forms and thus cannot unconditionally be determined, but it is not restricted to specific one inasmuch as the desired effect can be ensured. In particular, the agent preferably comprises maslinic acid, erythrodiol, ursolic acid, uvaol, betulinic acid and/or betulin. Specifically, the content is preferably not less than 0.0001% by mass, more preferably not less than 0.001% by mass, further preferably not less than 0.01% by mass, more preferably not less than 0.1% by mass, more preferably 0.0001 to 99.99% by mass, more preferably 0.001 to 99.99% by mass, further preferably 0.01 to 99.99% by mass, more preferably 0.1 to 99.99% by mass, more preferably 0.2 to 99.99% by mass, more preferably 0.5 to 99.99% by mass and more preferably 1 to 99.99% by mass, but the content is not limited to any specific range.

The concentrations of maslinic acid, erythrodiol, ursolic acid, uvaol, betulinic acid, betulin and physiologically acceptable salts thereof or derivatives thereof to be incorporated into the antitumor agent, tumor cell-proliferation-inhibitory agent, tumor cell-killing agent or tumor cell-metastasis-inhibitory agent according to the present invention as well as the amount of theantitumor agent may appropriately be determined depending on the purposes of using the same and the intensity of the required effects thereof.

When using maslinic acid, erythrodiol, ursolic acid, uvaol, betulinic acid, betulin and physiologically acceptable salts thereof or derivatives thereof as raw materials for theantitumor agent, the agent may comprise these compounds in an amount preferably ranges from 1 to 99.99% by mass, more preferably 10 to 99.99% by mass, more preferably 30 to 99.99% by mass, more preferably 50 to 99.99% by mass, more preferably 70 to 99.99% by mass and more preferably 90 to 99.99% by mass.

Moreover, the dose of maslinic acid, erythrodiol, ursolic acid, uvaol, betulinic acid, betulin and physiologically acceptable salts thereof or derivatives thereof to be incorporated into the antitumor agent, tumor cell-proliferation-inhibitory agent, tumor cell-killing agent or tumor cell-metastasis-inhibitory agent according to the present invention may vary depending on various conditions such as the species of the subject to which the agent is administered, age, sex, body weight, degree of symptoms and conditions of health and therefore, cannot unconditionally be determined. However, it is sufficient that the agent is orally or parenterally administered to adults at least one time per day in a dose of preferably not less than 0.1 mg, more preferably not less than 1 mg, more preferably 1 mg to 10000 mg, more preferably 10 mg to 10000 mg, more preferably 10 mg to 3000 mg, more preferably 100 mg to 1000 mg, more preferably 100 mg to 7500 mg, more preferably 200 mg to 5000 mg, more preferably 500 mg to 3000 mg, as expressed in terms of the amount of maslinic acid, erythrodiol, ursolic acid, uvaol, betulinic acid, betulin and physiologically acceptable salts thereof or derivatives thereof. If theantitumor agent of the present invention is an external preparation and if the agent is, for instance, applied in an amount of 10 g, theantitumor agent of the present invention comprising maslinic acid, erythrodiol, ursolic acid, uvaol, betulinic acid, betulin and physiologically acceptable salts thereof or derivatives thereof in an amount preferably ranging from 0.001 to 50% by mass, more preferably 0.001 to 30% by mass, more preferably 0.01 to 30% by mass, more preferably 0.01 to 10% by mass, more preferably 0.1 to 1% by mass, more preferably 0.1 to 20% by mass, more preferably 0.2 to 15% by mass, more preferably 0.5 to 10% by mass and more preferably 1 to 5% by mass at a frequency of at least one time per day, but the present invention is not restricted to this specific embodiment.

The antitumor agent of the present invention comprises, as effective components, maslinic acid, erythrodiol, ursolic acid, uvaol, betulinic acid, betulin and physiologically acceptable salts thereof or derivatives thereof. Maslinic acid, erythrodiol, ursolic acid, uvaol, betulinic acid, betulin and physiologically acceptable salts thereof or derivatives thereof possess an excellent tumor cell-proliferation-inhibitory effect, an excellent tumor cell-killing effect and an excellent tumor cell-metastasis-inhibitory effect and therefore, the antitumor agent of the present invention can clinically be used in various places and one can thus enjoy the excellent antitumor effect of the agent. In addition, maslinic acid, erythrodiol, ursolic acid, uvaol, betulinic acid, betulin and physiologically acceptable salts thereof or salts of derivatives thereof incorporated into the antitumor agent of the present invention are preferably prepared using natural plant bodies as raw materials and therefore, the antitumor agent of the present invention has low toxicity and can safely be administered and the stable supply of the agent can be ensured. In particular, maslinic acid and/or physiologically acceptable salts thereof possess quite excellent tumor cell-proliferation-inhibitory, tumor cell-killing and tumor cell-metastasis-inhibitory effects and therefore, the antitumor agent of the present invention can clinically be used in various places and one can thus enjoy the excellent antitumor effect of the agent. Moreover, maslinic acid and/or physiologically

acceptable salts thereof are preferably obtained from a raw material or olive plants, which have long been used as foodstuffs and therefore, the antitumor agent of the present invention has quite low toxicity and can safely be administered and the stable supply of the agent can be ensured.

Examples

The present invention will hereunder be described in more detail with reference to the following working Examples, but the present invention is not restricted to these specific Examples at all.

Erythrodiol, ursolic acid, uvaol, betulinic acid and betulin as 5-membered ring-containing triterpenes used in the following Examples were purchased as reagents. The reagents of HPLC grade were used without any pre-treatment and other reagents were dissolved in ethanol heated to the boiling point thereof till the saturation point, followed by cooling the saturated solution to thus recrystallized the reagents, separation through filtration and evaporation to dryness to give purified products. Maslinic acid was extracted from olive plants, purified and used after confirming whether the purity thereof was 95%. The details of the procedures for extraction and/or isolation of maslinic acid will be described below, while giving an example.

[Preparation Example 1]

Dried fruits (including seeds) derived from homegrown olive plant (Olea europaea L.) (1 kg) were crushed or pulverized and then 3 L of hexane was added to the crushed product and the extraction was continued over 3 hours. Then the extraction procedure was repeated 4 times to give defatted fruits, the seeds were removed from the defatted product, pulverized and again extracted with 5 volumes of hexane over 3 hours to thus obtain 229 g of defatted lees from which oil components were completely removed. To the defatted lees, there were added 10 volumes of an aqueous ethanol solution having an ethanol content of 60% by mass and the extraction was continued over 3 hours with vigorous stirring at room temperature. The whole system was filtered and the resulting filtrate was concentrated to dryness to give 112.7 g of an extract.

To 100 g of the extract, there was added 2 L of water followed by vigorous stirring of the resulting mixture at room temperature for one hour. The whole system was treated by centrifugation, the resulting supernatant was removed through decantation and the remaining precipitates were dried to give 10.0 g of a concentrate.

Then the concentrate was fractionated by the silica gel column chromatography in which a column packed with about 40 volumes (400 g) of silica gel was used. First, an eluting solution or a 3:1 hexane: ethyl acetate mixture was passed through the column in an amount of 10 times (4000 mL) the volume of the packed silica gel to thus elute all sorts of undesirable components and then an eluting solution or a 1:1 hexane: ethyl acetate mixture was passed through the column in an amount of 2.5 times (1000 mL) the volume of the silica gel to thus elute all sorts of undesirable components. Subsequently, the intended maslinic acid was eluted by passing an eluting solution or a 1:1 hexane: ethyl acetate mixture through the silica gel column in an amount of 10 times (4000 mL) the volume of the packed silica gel to thus give a crude maslinic acid-containing fraction. After removing the hexane and ethyl acetate, the fraction was dried in a vacuum to give 1.96 g of a maslinic acid-containing fractionated product.

Moreover, this crude maslinic acid-containing fractionated product was purified by the ODS column chromatography using a column packed with octadecyl silica gel in an amount of about 30 times (60 g) the volume of the product. First, an eluting solution or a 8:2 methanol: water mixture was passed through the column in an amount of 10 times the volume of the packed gel (600 mL) to thus elute all sorts of undesirable components. Then the target maslinic acid was eluted by passing an eluting solution or an 8:2 methanol: water mixture in an amount of 30 times the volume of the packed column (1800 mL) to thus give a purified maslinic acid-containing fractionated product. After the removal of the methanol, the maslinic acid-containing fraction was dried in a vacuum to give 1.51 g of purified maslinic acid 1.

At this stage, the purified maslinic acid 1 was analyzed by NMR, MS and GC techniques and it was confirmed that a part of the purified maslinic acid was in sodium and potassium salts and the remaining majority thereof was in its free state and that the purity thereof as maslinic acid was not less than 95%.

[Preparation Example 2]

Olive (Olea europaea L.) of Italy growth was subjected to oil expression procedures to give 500 g of a residue obtained after the oil expression, followed by the addition of 10 volumes of an aqueous ethanol solution having an ethanol content of 65% by mass and subsequent extraction at room temperature for 3 hours with vigorous stirring. The whole volume of the extraction system was filtered and then the resulting filtrate was concentrated to dryness to give 20.2 g of an extracted substance.

To this extracted substance, there were added 1 L of n-butanol and 1 L of water, the resulting mixture was stirred for 10 minutes and then the mixture was separated into an n-butanol phase and an aqueous phase. After the

removal of the n-butanol from the n-butanol phase, the residue thus obtained was dried in a vacuum to give 13.3 g of a concentrate.

Then the concentrate was fractionated by the silica gel column chromatography using a column packed with about 40 volumes (500 g) of silica gel. First, an eluting solution or a 3:1 hexane: ethyl acetate mixture was passed through the column in an amount of 10 times (5000 mL) the volume of the packed silica gel to thus elute all sorts of undesirable components and then an eluting solution or a 1:1 hexane: ethyl acetate mixture was passed through the column in an amount of 2.5 times (1250 mL) the volume of the packed silica gel to thus elute all sorts of undesirable components. Subsequently, the target maslinic acid was eluted by passing an eluting solution or a 1:1 hexane: ethyl acetate mixture through the silica gel column in an amount of 10 times (5000 mL) the volume of the packed silica gel to thus give a crude maslinic acid-containing fraction. After removing the hexane and ethyl acetate, the fraction was dried in a vacuum to give 2.66 g of a maslinic acid-containing fractionated product.

Moreover, this crude maslinic acid-containing fractionated product was purified by the ODS column chromatography using a column packed with octadecyl silica gel in an amount of about 30 times (80 g) the volume of the product. First, an eluting solution or a 8:2 methanol: water mixture was passed through the column in an amount of 10 times the volume of the packed gel (800 mL) to thus elute all sorts of undesirable components. Then the target maslinic acid was eluted by passing an eluting solution or an 8:2 methanol: water mixture in an amount of 30 times the volume of the packed column (2400 mL) to thus give a purified maslinic acid-containing fraction. After the removal of the methanol, the maslinic acid-containing fraction was dried in a vacuum to give 2.06 g of purified maslinic acid 2.

At this stage, the purified maslinic acid 2 was analyzed by NMR, MS and GC techniques and it was confirmed that a part of the purified maslinic acid was in its free state and the remaining majority thereof was in sodium and potassium salts and that the purity thereof as maslinic acid was not less than 97%.

Maslinic acid, erythrodiol, ursolic acid, uvaol, betulinic acid and betulin derivatives were prepared as follows:

[Synthetic Example 1]: Ethyl Maslinate

To 50 mL of chloroform, there were dissolved 4.5 g of maslinic acid and 1.0 g of triethylamine, separately 1.1 g of thionyl chloride was dissolved in 10 mL of chloroform to give a thionyl chloride solution and then the maslinic acid solution was stirred for one hour under cooling with ice, while dropwise adding the thionyl chloride solution to the maslinic acid solution. Then 3.2 g of ethanol was added to the reaction system and the resulting mixture was stirred for 3 hours under cooling with ice, while dropwise addition of a solution of 1.0 g of triethylamine in 10 mL of chloroform. After the completion of the reaction, the components soluble in chloroform were extracted, the chloroform was distilled off to give a crude reaction product and the latter was purified by the silica gel chromatography to thus recover 3.5 g of maslinic acid ethyl ester.

[Synthetic Example 2]: 2,3-O-di-acetyl-maslinic acid

Maslinic acid (2.0 g) was dissolved in 100 mL of pyridine, 50 mL of acetic acid anhydride was added to the resulting solution and the mixture was stirred overnight. After the pyridine and the acetic acid anhydride were distilled off, the residue obtained was dissolved in ether, this ether phase was washed once with a 1N hydrochloric acid aqueous solution, once with a saturated sodium hydrogen carbonate aqueous solution and three times with pure water, magnesium sulfate was added to the ether phase and then the mixture was allowed to stand overnight. The magnesium sulfate was removed from the mixture through filtration, the ether was distilled off and then the resulting crude reaction product was purified by the silica gel column chromatography to give 2.2 g of 2,3-O-di-acetyl-maslinic acid.

[Synthetic Example 3]: 2,3-O-di-triethylsilyl-maslinic acid triethylsilyl ester

Maslinic acid (1.0 g ) was dissolved in 200 mL of anhydrous dimethylformamide, 144.0 mg of imidazole and 350 µL of triethylsilyl chloride were added to the resulting solution at 0°C, the reaction container was tightly sealed and the content thereof was stirred for 2 hours. After the dimethylformamide was distilled off, the resulting residue was dissolved in ether, the ether phase was washed once with a 1N hydrochloric acid aqueous solution, once with a saturated sodium hydrogen carbonate aqueous solution and three times with pure water, magnesium sulfate was added to the ether phase and then the mixture was allowed to stand overnight. The magnesium sulfate was removed from the mixture through filtration, the ether was distilled off and then the resulting crude reaction product was purified by the silica gel column chromatography to give 1.5 g of 2,3-O-di-triethylsilyl-maslinic acid triethylsilyl ester.

[Synthetic Example 4]: 2,3-O-di-stearoyl-maslinic acid ethyl ester

The maslinic acid ethyl ester (1.0 g) prepared in Synthetic Example 1 was dissolved in 50 mL of toluene, 5.0

g of triethylamine was added to the resulting solution, further 6.0 g of stearic acid chloride was gradually added to the solution under cooling with ice, the resulting mixture was stirred for one hour and further stirred over 9 hours, while the temperature of the mixture was gradually reduced back to room temperature. A sufficient amount of a 1N hydrochloric acid aqueous solution was added to the mixture, followed by extraction with ether, washing the ether phase once with a saturated sodium hydrogen carbonate aqueous solution and three times with pure water, magnesium sulfate was added to the ether phase and then the mixture was allowed to stand overnight. The magnesium sulfate was removed from the mixture through filtration, the ether was distilled off and then the resulting crude reaction product was purified by the silica gel column chromatography to give 1.2 g of 2,3-O-di-stearoyl- maslinic acid ethyl ester.

[Synthetic Example 5]: 3,28-O-di-acetyl-erythrodiol

Erythrodiol (5.0 g) was dissolved in 250 mL of pyridine, 100 mL of acetic acid anhydride was added to the resulting solution and the mixture was allowed to stand overnight. After the pyridine and the acetic acid anhydride were distilled off, the residue thus obtained was dissolved in ether, the resulting ether phase was washed once with a 1N hydrochloric acid aqueous solution, once with a saturated sodium hydrogen carbonate aqueous solution and three times with pure water, magnesium sulfate was added to the ether phase and then the mixture was allowed to stand overnight. The magnesium sulfate was removed from the mixture through filtration, the ether was distilled off and then the resulting crude reaction product was purified by the silica gel column chromatography to give 5.4 g of 3,28-O-di-acetyl-erythrodiol.

[Synthetic Example 6]: ursolic acid ethyl ester

To 50 ml of chloroform, there were dissolved 5.0 g of ursolic acid and 1.1 g of triethylamine and then stirred for one hour under cooling with ice while dropwise adding a solution of 1.2 g of thionyl chloride in 10 mL of chloroform to the resulting solution. Subsequently, 3.5 g of ethanol was added to the solution and stirred for 3 hours under cooling with ice while dropwise adding a solution of 1.1 g of triethylamine in 10 mL of chloroform to the reaction solution. After the completion of the reaction, the components soluble in chloroform were removed through chloroform extraction, the chloroform was distilled off and the resulting crude reaction product was purified by the silica gel column chromatography to thus recover 3.8 g of ursolic acid ethyl ester.

[Synthetic Example 7]: 3,28-O-di-acetyl-uvaol

Uvaol (5.0 g) was dissolved in 250 mL of pyridine, 100 mL of acetic acid anhydride was added to the resulting solution and then the mixture was allowed to stand overnight. After the pyridine and the acetic anhydride were distilled off, the resulting residue was dissolved in ether, the resulting ether phase was washed once with a 1N hydrochloric acid aqueous solution, once with a saturated sodium hydrogen carbonate aqueous solution and three times with pure water, magnesium sulfate was added to the ether phase and then the mixture was allowed to stand overnight. The magnesium sulfate was removed from the mixture through filtration, the ether was distilled off and then the resulting crude reaction product was purified by the silica gel column chromatography to give 5.4 g of 3,28-O-di-acetyl-uvaol.

[Synthetic Example 8]: betulinic acid ethyl ester

To 50 ml of chloroform, there were dissolved 5.0 g of betulinic acid and 1.1 g of triethylamine and then stirred for one hour under cooling with ice while dropwise adding a solution of 1.2 g of thionyl chloride in 10 mL of chloroform to the resulting solution. Subsequently, 3.5 g of ethanol was added to the solution and stirred for 3 hours under cooling with ice while dropwise adding a solution of 1.1 g of triethylamine in 10 mL of chloroform to the reaction solution. After the completion of the reaction, the components soluble in chloroform were removed through chloroform extraction, the chloroform was distilled off from the extract and the resulting crude reaction product was purified by the silica gel column chromatography to thus recover 3.8 g of betulinic acid ethyl ester.

[Synthetic Example 9]: 3,28-O-di-acetyl-betulin

Betulin (5.0 g) was dissolved in 250 mL of pyridine, 100 mL of acetic acid anhydride was added to the resulting solution and then the mixture was allowed to stand overnight. After the pyridine and the acetic anhydride were distilled off, the resulting residue was dissolved in ether, the resulting ether phase was washed once with a 1N hydrochloric acid aqueous solution, once with a saturated sodium hydrogen carbonate aqueous solution and three times with pure water, magnesium sulfate was added to the ether phase and then the mixture was allowed to stand overnight. The magnesium sulfate was removed from the mixture through filtration, the ether was distilled off from the ether phase

and then the resulting crude reaction product was purified by the silica gel column chromatography to give 5.4 g of 3,28-O-di-acetyl- betulin.

[Test Example 1]: Tests for Tumor Cell-Proliferation-Inhibitory Effect and for Tumor Cell-Extinction

Tests for determining the tumor cell-proliferation-inhibitory effect and the tumor cell-killing effect were conducted according to the following method. To a 6-well plate, a culture medium was dispensed in an amount of 2 mL/well, B-16 melanoma cells were then inoculated in a desired quantity and thereafter the melanoma cells were cultivated by allowing the plate or the culture medium to stand at 37°C and a carbon dioxide concentration of 5%. On the following day, a test sample solution prepared was added to and admixed with the content of each well to a desired concentration and the cultivation of the melanoma cells was continued. The culture medium was then exchanged on the $5^{th}$ day from the initiation of the cultivation and the test sample solution was again added to each well. On the next day, the culture medium was removed to recover the cultivated melanoma cells, followed by washing the cells with PBS (phosphate buffered saline), determination of the viable cell count, and then calculation of the cell growth rate according to the following equation 1. The tumor cell-proliferation-inhibitory effect and the tumor cell-killing effect of each test sample were evaluated on the basis of the cell growth rate thus determined. The results were compared with the cell growth rate observed when any test sample was not added (control).

$$\text{Cell Growth Rate}(\%) = (A/B) \times 100$$

Wherein A: the viable cell count observed when each test sample was added and B: the viable cell count observed for the control.

The foregoing effects were evaluated on the basis of the cell growth rate observed when melanoma cells were cultivated while adding maslinic acid, erythrodiol, ursolic acid, uvaol, betulinic acid, betulin and physiologically acceptable salts thereof or derivatives thereof to each culture medium in a concentration as specified in the following Table 1. As comparative examples, oleanolic acid, β-amyrin, α-amyrin and lupeol were added to the culture medium to thus determine and evaluate the cell growth rate by the same method used above. The results thus obtained are summarized in the following Table 1.

Table 1

| | Concentration (ppm) | | | | | | | |
|---|---|---|---|---|---|---|---|---|
| | 5 | 10 | 15 | 20 | 25 | 30 | 40 | 50 |
| Oleanolic acid* | | | 100 | 100 | 100 | 100 | 100 | 94 |
| β-Amyrin* | | | 100 | 100 | 100 | 100 | 100 | 100 |
| α-Amyrin* | | | 100 | 100 | 100 | 100 | 100 | 100 |
| Lupeol* | | | 100 | 100 | 100 | 100 | 100 | 100 |
| Purified maslinic acid 1 | 67 | 43 | 6 | 0 | 0 | | | |
| Purified maslinic acid 2 | 75 | 47 | 11 | 0 | 0 | | | |
| Erythrodiol | 73 | | 69 | | 53 | | | 43 |
| Ursolic acid | 64 | 40 | 3 | 0 | 0 | | | 0 |
| Uvaol | 80 | | 69 | | 49 | | | 30 |
| Betulinic acid | 88 | | 77 | | 58 | | | 38 |
| Betulin | 100 | | 78 | | 72 | | | 63 |
| Comp. Of Synthetic Ex. 1 | 73 | 49 | 18 | 0 | 0 | | | |
| Comp. Of Synthetic Ex. 2 | 89 | 63 | 34 | 4 | 0 | | | |
| Comp. Of Synthetic Ex. 3 | 93 | 75 | 54 | 21 | 0 | | | |
| Comp. Of Synthetic Ex. 4 | 100 | 82 | 52 | 13 | 0 | | | |

*: Comparative Sample

Table 1 (continued)

| | Concentration (ppm) | | | | | | | |
|---|---|---|---|---|---|---|---|---|
| | 5 | 10 | 15 | 20 | 25 | 30 | 40 | 50 |
| Comp. Of Synthetic Ex. 5 | 68 | 50 | 23 | 0 | 0 | | | |
| Comp. Of Synthetic Ex. 6 | 79 | | 72 | | 58 | | | 47 |
| Comp. Of Synthetic Ex. 7 | 85 | | 71 | | 54 | | | 42 |
| Comp. Of Synthetic Ex. 8 | 96 | | 81 | | 69 | | | 53 |
| Comp. Of Synthetic Ex. 9 | 100 | | 79 | | 68 | | | 60 |

The results listed in Table 1 indicate that the comparative samples: β-amyrin, α-amyrin and lupeol do not possess any tumor cell-proliferation-inhibitory effect at all. In addition, oleanolic acid, which possesses a carcinogenic promoter (or tumor promoter)-inhibitory activity, shows quite weak tumor cell-proliferation-inhibitory effect only at a higher concentration. Contrary to this, maslinic acid, erythrodiol, ursolic acid, uvaol, betulinic acid, betulin and physiologically acceptable salts thereof or derivatives thereof were found to possess quite strong tumor cell-proliferation-inhibitory effects. In particular, maslinic acid, ursolic acid and physiologically acceptable salts thereof or derivatives thereof showed quite intensive tumor cell-killing effects at a concentration of not less than 2 times that required for showing the tumor cell-proliferation-inhibitory effects. Such an action was not observed for oleanolic acid, β-amyrin, α-amyrin and lupeol, at all.

From the foregoing, it was proved that according to the present invention, there can be provided anantitumor agent having quite excellent tumor cell-proliferation-inhibitory effect and tumor cell-killing effect.

[Test Example 2]: Test for Malignant Melanoma Metastasis-Inhibitory Effect

Tests for evaluating the malignant melanoma metastasis-inhibitory effect of each test sample were conducted according to the following method. Whister female rats (6-week-old; average body weight: 160 g) were preliminarily bred with a powdery feed preparation having a composition of AIN-93 for one week, these animals were divided into 5 groups (8 animals per group) so as to equalize the average body weight and then a previously prepared suspension of B16 melanoma cells was intravenously injected into each rat. Subsequently, these animals were bred with a powdery feed preparation having a composition of AIN-93 and cotton seed oil in which each of maslinic acid, erythrodiol, ursolic acid, uvaol, betulinic acid, betulin, compounds of Synthetic Examples 1 and 2,or oleanolic acid had been dissolved in a predetermined concentration was intraperitoneally injected into each animal or orally administered thereto using a sonde, every other day starting from the $2^{nd}$ day after the injection of the B16 melanoma cells. In this connection, only cotton seed oil was administered to the control group. On the $15^{th}$ day after the injection of the B16 melanoma cells, the lung of each animal was taken out, the lungs were examined to determine the number of cancer lesions thus metastasized and to thus calculate the rate of metastasis-inhibition in accordance with the following equation 2. The tumor cell-metastasis-inhibitory effect of each test sample could be evaluated on the basis of the resulting rate of metastasis-inhibition.

$$\text{Rate of Metastasis-Inhibition (\%)} = [(D - C)/D] \times 100$$

Wherein C: Average number of cancer lesions metastasized observed in each animal group; and D: average number of cancer lesions metastasized observed in the control group.

Maslinic acid, erythrodiol, ursolic acid, uvaol, betulinic acid, betulin and physiologically acceptable salts thereof or derivatives thereof as well as cotton seed oil as the control were inspected for the rate of metastasis-inhibition (RMI) using the dose and administration methods specified in the following Table 2. In addition, the same procedures used above were repeated except for using oleanolic acid as a comparative sample to thus evaluate the rate of metastasis-inhibition (RMI) thereof. The results thus obtained are summarized in the following Table 2.

Table 2

| Group No. | Substance Administered | Dose (mg/body wt. (kg)) | Adm. Method | RMI (%) |
|---|---|---|---|---|
| 1 | Cotton seed oil alone (control) | -- | Oral Adm.* | 0 |

*: Oral administration;

Table 2 (continued)

| Group No. | Substance Administered | Dose (mg/body wt. (kg)) | Adm. Method | RMI (%) |
|---|---|---|---|---|
| 2 | Oleanolic acid | 300 | Oral Adm. | 9.2 |
| 3 | Purified maslinic acid 1 | 50 | Oral Adm. | 61.4 |
| 4 | Purified maslinic acid 2 | 50 | I.P. Adm.** | 67.3 |
| 5 | Purified maslinic acid 2 | 100 | Oral Adm. | 78.6 |
| 6 | Erythrodiol | 100 | Oral Adm. | 56.3 |
| 7 | Ursolic acid | 100 | Oral Adm. | 79.5 |
| 8 | Uvaol | 100 | Oral Adm. | 43.7 |
| 9 | Betulinic acid | 100 | Oral Adm. | 51.2 |
| 10 | Betulin | 100 | Oral Adm. | 38.9 |
| 11 | Comp. Of Synthetic Ex. 1 | 100 | Oral Adm. | 77.6 |
| 12 | Comp. Of Synthetic Ex. 2 | 100 | Oral Adm. | 74.8 |

**: Intraperitoneal administration.

As will be clear from the data listed in the foregoing Table 2, maslinic acid, erythrodiol, ursolic acid, uvaol, betulinic acid, betulin and physiologically acceptable salts thereof or derivatives thereof could significantly ($P<0.05$) inhibit any metastasis of cancer to lung in both of the intraperitoneally administered group and the orally administered group as compared with the control group. Any significant effect was not observed when oleanolic acid was administered.

These results clearly indicate that maslinic acid, erythrodiol, ursolic acid, uvaol, betulinic acid, betulin and physiologically acceptable salts thereof or derivatives thereof possess quite effective or efficient tumor cell-metastasis-inhibitory effect.

| [Product Example 1]: Injection | |
|---|---|
| (1) Maslinic acid prepared in Preparation Example 1 | 10.0 mg |
| (2) Polyoxyethylene hardened castor oil | 200.0 mg |
| (3) Anhydrous ethanol | As much as suffices |

In accordance with the foregoing mixing rate, the component (1) was sufficiently admixed with the component (2) and then an appropriate amount of the component (3) was added to the foregoing mixture to adjust the total volume of the mixture to 1 mL and to thus give an injection. In this connection, this solution can be administered after the dilution with a proper amount of isotonic sodium chloride solution.

| [Product Example 2]: Tablet | |
|---|---|
| (1) Maslinic acid of Preparation Example 2 | 1.0 mg |
| (2) Lactose | 94.0 mg |
| (3) Corn starch | 34.0 mg |
| (4) Crystalline cellulose | 20.0 mg |
| (5) Magnesium stearate | 1.0 mg |

The foregoing components (1) to (4) were first sufficiently admixed together in the foregoing mixing ratio and then the component (5) was added and mixed together. The resulting mixture was compressed into a tablet.

Maslinic acid, erythrodiol, ursolic acid, uvaol, betulinic acid, betulin and physiologically acceptable salts thereof or derivatives thereof included in the antitumor agent of the present invention possess excellent tumor cell-proliferation-inhibitory, tumor cell-killing and tumor cell-metastasis-inhibitory effects. In particular, maslinic acid and/or physiologically acceptable salts thereof possess considerably excellent tumor cell-proliferation-inhibitory, tumor cell-killing and tumor cell-metastasis-inhibitory effects. Maslinic acid, erythrodiol, ursolic acid, uvaol, betulinic acid, betulin and physiologically acceptable salts thereof or derivatives thereof can be artificially prepared, but maslinic acid, erythrodiol, ursolic acid, uvaol, betulinic acid and betulin can likewise obtained from, for instance, natural plants and therefore, they may provide a quite safeantitumor agent. In particular, maslinic acid and/or physiologically acceptable salts thereof are components capable of being isolated from olive plants, which have long been used as foods and therefore, they can provide a quite desirable antitumor agent having a strong antitumor effect and high safety.

## Claims

**1.** An antitumor agent comprising, as an effective component, a compound selected from the group consisting of maslinic acid, erythrodiol, uvaol, betulinic acid, betulin and physiologically acceptable salts thereof or derivatives thereof.

**2.** A tumor cell-proliferation-inhibitory agent comprising, as an effective component, a compound selected from the group consisting of maslinic acid, erythrodiol, ursolic acid, uvaol, betulinic acid, betulin and physiologically acceptable salts thereof or derivatives thereof.

**3.** A tumor cell-killing agent comprising, as an effective component, a compound selected from the group consisting of maslinic acid, erythrodiol, ursolic acid, uvaol, betulinic acid, betulin and physiologically acceptable salts thereof or derivatives thereof.

**4.** A tumor cell-metastasis-inhibitory agent comprising, as an effective component, a compound selected from the group consisting of maslinic acid, erythrodiol, uvaol, betulinic acid, betulin and physiologically acceptable salts thereof or derivatives thereof.

**5.** An antitumor agent comprising, as an effective component, maslinic acid and/or physiologically acceptable salts thereof.

**6.** A tumor cell-proliferation-inhibitory agent comprising, as an effective component, maslinic acid and/or physiologically acceptable salts thereof.

**7.** A tumor cell-killing agent comprising, as an effective component, maslinic acid and/or physiologically acceptable salts thereof.

**8.** A tumor cell-metastasis-inhibitory agent comprising, as an effective component, maslinic acid and/or physiologically acceptable salts thereof.

**9.** A method of using one or at least two members selected from the group consisting of maslinic acid, erythrodiol, uvaol, betulinic acid, betulin and physiologically acceptable salts thereof or derivatives thereof for achieving at least one effect selected from the group consisting of tumor cell-proliferation-inhibitory, tumor cell-killing and tumor cell-metastasis-inhibitory effects.

**10.** A tumor cell-proliferation-inhibitory agent comprising, as an effective component, ursolic acid and physiologically acceptable salts thereof or derivatives thereof.

**11.** A tumor cell-killing agent comprising, as an effective component, ursolic acid and physiologically acceptable salts thereof or derivatives thereof.

**12.** A method of using a compound selected from the group consisting of ursolic acid and physiologically acceptable salts thereof or derivatives thereof as a tumor cell-proliferation-inhibitory agent or a tumor cell-killing agent.

**13.** An antitumor agent comprising a compound selected from the group consisting of maslinic acid, erythrodiol, uvaol, betulinic acid, betulin and physiologically acceptable salts thereof or derivatives thereof; and a carrier and/or a diluent.

**14.** A raw material for an antitumor agent, comprising a compound selected from the group consisting of maslinic acid, erythrodiol, ursolic acid, uvaol, betulinic acid, betulin and physiologically acceptable salts thereof or derivatives thereof.

**15.** A method of using a compound selected from the group consisting of maslinic acid, erythrodiol, ursolic acid, uvaol, betulinic acid, betulin and physiologically acceptable salts thereof or derivatives thereof, as a raw material for an antitumor agent.

**16.** The antitumor agent as set forth in claim 1 wherein the compound selected from the group consisting of maslinic acid, erythrodiol, uvaol, betulinic acid, betulin and physiologically acceptable salts thereof or derivatives thereof is one extracted from a natural raw material.

**17.** The tumor cell-proliferation-inhibitory agent as set forth in claim 2 wherein the compound selected from the group consisting of maslinic acid, erythrodiol, ursolic acid, uvaol, betulinic acid, betulin and physiologically acceptable salts thereof or derivatives thereof is one extracted from a natural raw material.

**18.** The tumor cell-killing agent as set forth in claim 3 wherein the compound selected from the group consisting of maslinic acid, erythrodiol, ursolic acid, uvaol, betulinic acid, betulin and physiologically acceptable salts thereof or derivatives thereof is one extracted from a natural raw material.

**19.** The tumor cell-metastasis-inhibitory agent as set forth in claim 4 wherein the compound selected from the group consisting of maslinic acid, erythrodiol, uvaol, betulinic acid, betulin and physiologically acceptable salts thereof or derivatives thereof is one extracted from a natural raw material.

**20.** Use of a compound selected from the group consisting of maslinic acid, erythrodiol, uvaol, betulinic acid, betulin and physiologically acceptable salts thereof or derivatives thereof for the preparation of an antitumor agent.

**21.** Use of a compound selected from the group consisting of maslinic acid, erythrodiol, ursolic acid, uvaol, betulinic acid, betulin and physiologically acceptable salts thereof or derivatives thereof for the preparation of a tumor cell-proliferation-inhibitory agent.

**22.** Use of a compound selected from the group consisting of maslinic acid, erythrodiol, ursolic acid, uvaol, betulinic acid, betulin and physiologically acceptable salts thereof or derivatives thereof for the preparation of a tumor cell-killing agent.

**23.** Use of a compound selected from the group consisting of maslinic acid, erythrodiol, uvaol, betulinic acid, betulin and physiologically acceptable salts thereof or derivatives thereof for the preparation of a tumor cell-metastasis-inhibitory agent.

**24.** A method for inhibiting tumor cell-proliferation comprising the step of bringing a compound selected from the group consisting of maslinic acid, erythrodiol, ursolic acid, uvaol, betulinic acid, betulin and physiologically acceptable salts thereof or derivatives thereof into contact with tumor cells.

**25.** A method for killing tumor cells comprising the step of bringing a compound selected from the group consisting of maslinic acid, erythrodiol, ursolic acid, uvaol, betulinic acid, betulin and physiologically acceptable salts thereof or derivatives thereof into contact with tumor cells.

**26.** A method for inhibiting tumor cell-metastasis comprising the step of bringing a compound selected from the group consisting of maslinic acid, erythrodiol, uvaol, betulinic acid, betulin and physiologically acceptable salts thereof or derivatives thereof into contact with tumor cells.

**27.** A method for inhibiting tumor cell-proliferation comprising the step of incorporating, into tumor cells, a compound selected from the group consisting of maslinic acid, erythrodiol, ursolic acid, uvaol, betulinic acid, betulin and physiologically acceptable salts thereof or derivatives thereof.

**28.** A method for killing tumor cells comprising the step of incorporating, into tumor cells, a compound selected from the group consisting of maslinic acid, erythrodiol, ursolic acid, uvaol, betulinic acid, betulin and physiologically acceptable salts thereof or derivatives thereof.

**29.** A method for inhibiting tumor cell-metastasis comprising the step of incorporating, into tumor cells, a compound selected from the group consisting of maslinic acid, erythrodiol, uvaol, betulinic acid, betulin and physiologically acceptable salts thereof or derivatives thereof.

# INTERNATIONAL SEARCH REPORT

International application No.
PCT/JP01/06393

A. CLASSIFICATION OF SUBJECT MATTER
Int.Cl[7] A61K31/56, A61K35/78, A61P35/00, 04, C07J53/00

According to International Patent Classification (IPC) or to both national classification and IPC

B. FIELDS SEARCHED

Minimum documentation searched (classification system followed by classification symbols)
Int.Cl[7] A61K31/56-593, A61K35/78, A61P35/00-04, C07J53/00

Documentation searched other than minimum documentation to the extent that such documents are included in the fields searched

Electronic data base consulted during the international search (name of data base and, where practicable, search terms used)
CAPLUS (STN), REGISTRY (STN), WPI/L (DIALOG)

C. DOCUMENTS CONSIDERED TO BE RELEVANT

| Category* | Citation of document, with indication, where appropriate, of the relevant passages | Relevant to claim No. |
|---|---|---|
| X | Ken YASUKAWA, et al., "Antitumor-promoting activities of sterols and triterpenoids", Nippon Yuka Gakkai-shi, Vol.49, No.6, 20 June, 2000 (20.06.00), pages 571 to 583,<br>See page 578, table 2; pages 579, 580, table 4, on trout phosphoric acid, erythro-diol, uvaol, betulin acid, betulin, Ursolic acid | 1-8,10-11, 13-23 |
| X | Young-Kyoon Kim, et al., "Cytotoxic triterpenes from stem bark of Physocarpus intermedius", Planta Med., Vol.66, No.5, June, 2000, pages 485 to 486<br>See page 485, chemical compound 1, 2, 7; page 486, table 1 etc. , on trout phosphoric acid, betulin acid, ursolic acid | 1-8,10-11, 13-23 |
| X | US 5985924 A (JCR Pharmaceuticals Co., Ltd.), 16 November, 1999 (16.11.99),<br>See column 4, table 2 etc., on ursolic acid<br>& EP 774255 A1 & JP 9-143076 A | 2-4,10-11, 14-15,17-18, 21-23 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 October, 2001 (12.10.01) | 06 November, 2001 (06.11.01) |
| Name and mailing address of the ISA/<br>Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP01/06393

C (Continuation). DOCUMENTS CONSIDERED TO BE RELEVANT

| Category* | Citation of document, with indication, where appropriate, of the relevant passages | Relevant to claim No. |
|---|---|---|
| X | D. Es-Saddy, et al., "Effects of ursolic acid and its analogues on soybean 15-lipoxigenase activity and the proliferation rate of a human gastric tumor cell line", Mediators of Inflammation, Vol.3, (1994), pages 181 to 184<br>See page 183, Figs. 3, 4 etc., on ursolic acid, uvaol | 1-4,10-11, 13-23 |
| X | JP 63-60949 A (Rikagaku Kenkyusho), 17 March, 1988 (17.03.88) (Family: none)<br>See pages 6 to 7, on ursolic acid, uvaol | 1-4,10-11, 13-23 |
| X | JP 63-57519 A (Goshi Kaisha Minoph), 12 March, 1988 (12.03.88) (Family: none)<br>See pages 4 to 5, working examples II, etc., on erythro-diol | 1-4,13-23 |
| X | JP 1-143832 A (Toa Nenryo Kogyo K.K.), 06 June, 1989 (06.06.89) (Family: none)<br>See Full text, on betulin | 1-4,13-23 |
| X | US 5962527 A (University of Illinois Foundation), 05 October, 1999 (05.10.99),<br>See Full text, on betulin acid<br>& WO 96/29068 A2 & EP 814795 A1<br>& JP 11-502525 A | 1-4,13-23 |

Form PCT/ISA/210 (continuation of second sheet) (July 1992)

**INTERNATIONAL SEARCH REPORT**

International application No.
PCT/JP01/06393

**Box I Observations where certain claims were found unsearchable (Continuation of item 1 of first sheet)**

This international search report has not been established in respect of certain claims under Article 17(2)(a) for the following reasons:

1. ☒ Claims Nos.: 9,12,24-29
because they relate to subject matter not required to be searched by this Authority, namely:

Claims 9, 12 and 24 to 29 pertain to methods for treatment of the human body by therapy and thus relate to a subject matter which this International Searching Authority is not required, under the provisions of Article 17(2)(a)(i) of the PCT and Rule 39.1(iv) of the Regulations under the PCT, to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)